(19) Europäisches Patentamt
European Patent Office
Office européen des brevets

(11) **EP 2 018 418 B1**

(12) **EUROPEAN PATENT SPECIFICATION**

(45) Date of publication and mention of the grant of the patent:
**10.12.2014 Bulletin 2014/50**

(51) Int Cl.:
**C12N 5/00** *(2006.01)*

(21) Application number: **07732504.1**

(22) Date of filing: **24.04.2007**

(86) International application number:
**PCT/GB2007/001464**

(87) International publication number:
**WO 2007/125288 (08.11.2007 Gazette 2007/45)**

(54) **SUBSTRATE FOR THE GROWTH OF CULTURED CELLS IN THREE DIMENSIONS**

SUBSTRAT FÜR DAS WACHSTUM KULTIVIERTER ZELLEN IN DREI DIMENSIONEN

SUBSTRAT PERMETTANT LA CROISSANCE DE CELLULES CULTIVÉES DANS TROIS DIMENSIONS

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **28.04.2006 GB 0608403**
**20.05.2006 GB 0610120**
**12.01.2007 GB 0700592**

(43) Date of publication of application:
**28.01.2009 Bulletin 2009/05**

(73) Proprietor: **REINNERVATE LIMITED**
**Old Elvet**
**Durham DH1 3HP (GB)**

(72) Inventors:
• **PRZYBORSKI, Stefan Alexander**
**Durham DH1 3LE (GB)**
• **CAMERON, Neil**
**Durham DH1 3LE (GB)**

(74) Representative: **Docherty, Robert Charles**
**Harrison IP**
**1st Floor**
**Box Tree House**
**Northminster Business Park**
**Northfield Lane**
**York, YO26 6QU (GB)**

(56) References cited:
**EP-A1- 0 322 212     WO-A-00/34454**
**WO-A1-99/09149      US-A- 5 266 476**
**US-B1- 6 281 257**

• **HAYMAN M W ET AL: "Growth of human stem cell-derived neurons on solid three-dimensional polymers" JOURNAL OF BIOCHEMICAL AND BIOPHYSICAL METHODS, AMSTERDAM, NL, vol. 62, no. 3, 31 March 2005 (2005-03-31), pages 231-240, XP004759810 ISSN: 0165-022X**

**Description**

The invention relates to a cell culture substrate comprising a polymerised high internal phase emulsion polymer (polyHIPE) adapted for installation and use in existing cell culture plastic-ware for the growth of cells, typically mammalian cells and the use of the substrate in a cell culture system for analysis of proliferation, differentiation and function of cells.

The culturing of eukaryotic cells, for example mammalian cells, has become a routine procedure and cell culture conditions which allow cells to proliferate, differentiate and function are well defined. Typically, cell culture of mammalian cells requires a sterile vessel, usually manufactured from plastics (typically polystyrene), defined growth medium and, in some examples, feeder cells and serum, typically calf serum. The feeder cells function to provide signals which stimulate cell proliferation and/or maintain cells in an undifferentiated state and can influence cell function. The culturing of prokaryotic cells, for example bacterial cells is also an established technique and has been used for many years for the production of valuable molecules.

The culturing of mammalian cells has many applications and there are numerous in vitro assays and models where cell culture is used for experimentation and research; for example the use of cells in tissue engineering; the use of mammalian expression systems for the production of recombinant protein and the use of mammalian cells in the initial screening of drugs.

Tissue engineering is a science which has implications with respect to many areas of clinical and cosmetic surgery. More particularly, tissue engineering relates to the replacement and/or restoration and/or repair of damaged and/or diseased tissues to return the tissue and/or organ to a functional state. For example, tissue engineering is useful in the provision of skin grafts to repair wounds occurring as a consequence of: contusions, or burns, or failure of tissue to heal due to venous or diabetic ulcers. Tissue engineering requires *in vitro* culturing of replacement tissue followed by surgical application of the tissue to a wound to be repaired.

The production of recombinant protein in cell expression systems is based either on prokaryotic cell expression or eukaryotic cell expression. The latter is preferred when post-translation modifications to the protein are required. Eukaryotic systems include the use of mammalian cells, e.g. Chinese Hamster Ovary cells; insect cells e.g. *Spodoptera spp;* or yeast e.g. *Saccharomyces spp, Pichia spp.* The large scale production of recombinant proteins requires a high standard of quality control since many of these proteins are used as pharmaceuticals, for example: growth hormone; leptin; erythropoietin; prolactin; TNF, interleukins; granulocyte colony stimulating factor (G-CSF); granulocyte macrophage colony stimulating factor (GM-CSF); ciliary neurotrophic factor (CNTF); cardiotrophin-1 (CT-1); leukemia inhibitory factor (LIF); oncostatin M (OSM); interferon, IFN$\alpha$, IFN$\gamma$. Moreover, the development of vaccines, particularly subunit vaccines, (vaccines based on a defined antigen, for example gp120 of HIV), requires the production of large amounts of pure protein free from contaminating antigens which may provoke anaphylaxis. In some situations it is desirable to manufacture recombinant protein in cells that are differentiated and able to process the expressed polypeptide. Post-translation processing includes the proteolytic processing of precursor proteins and the addition or removal of chemical groups (e.g. phosphorylation, prenylation, glucosylation, farnesylation).

Moreover, mammalian cells are used in initial drug screening to determine whether a lead therapeutic (e.g. a small molecule agonist or antagonist, a monoclonal antibody, peptide therapeutic, nucleic acid aptamer, small inhibitory RNA (siRNA)) has efficacy before animal trials are undertaken.

There is a need to provide improved cell culture systems in which mammalian cells can be cultured to provide a population of cells that are as far as technically possible close to their natural state to enable the analysis of cell proliferation, differentiation and function in a reliable manner.

Cell culture systems are known in the art and have been available to the skilled person for many years. Cell culture typically involves the growth of cells in monolayer culture under sterile conditions in closed cell culture vessels. More recently cell culture systems have been developed that provide means by which cells can be cultured in 3 dimensions to more closely resemble the situation found *in vivo.* For example, WO2003/014334 discloses an *in vitro* cell culture method which provides a culture regime that allows prostate epithelial cells to form prostate-like-acini which closely resemble prostate acini found *in vivo.* These have utility in testing the efficacy of anti cancer agents with respect to controlling proliferation or metastasis of prostate cancer cells since transformed prostate epithelial cells also form acini in the cell culture system.

Furthermore, cell culture substrates are described in WO00/34454, which comprises microcellular polymeric materials which are described as polyHIPE polymers. These polymers form reticulate structures of pores that interconnect with one another to provide a substrate to which cells can attach and proliferate. The process for the formation of polyHIPEs allows pore volume to be accurately controlled with pore volume varying from 75% to 97%. Pore sizes can vary between 0.1 to 1000 micron and the diameter of the interconnecting members from a few microns to 100 microns. Furthermore the polyHIPEs can be combined with additional components that facilitate cell proliferation and/or differentiation. PolyHIPEs are therefore versatile substrates on which cells can attach and proliferate in a cell culture system. Processes for the preparation of polyHIPEs are well known in the art and also disclosed in WO2004/005355 and WO2004/004880.

PolyHIPEs are commercially available and comprise for example oil phase monomers styrene, divinyl benzene and a surfactant, for example Span 80 sorbitan monooleate. In addition, the rigidity of the polymer formed during processing of the polyHIPE may be affected by the inclusion of a monomer such as 2-ethylhexyl acrylate. The process for the formation of polyHIPE from an emulsion is initiated by the addition of a catalyst such as ammonium persulphate.

The processes for the manufacture of polyHIPEs in WO00/34454, WO2004/005355 and WO2004/004880 describe various conditions for the formation polymers. For example, styrene concentration can vary from 15 %(w/w) to 78 % (w/w); surfactant concentration varies between 14 %(w/w) and 15 %(w/w) and the addition of the monomer 2-ethylhexyl acrylate varies between 60 %(w/w) and 62 %(w/w). Moreover, the disclosures in these applications relate to the production of unitary cell supports to which cells attach and grow. The resultant polyHIPEs formed by these processes have pore volumes that vary from 75% to 97%.

We herein describe a process for the formation of a polyHIPE that has superior properties specifically designed for the routine culture of cells, typically mammalian cells, when compared to polyHIPEs formed by prior art processes. The polyHIPEs thus formed have a porosity of around 90% and are further processed into thin membranes or layers (for example, by microtome sectioning) to produce a cell culture substrate comprising a plurality of thin polyHIPE adapted to fit existing cell culture vessels. The polyHIPE is also modified by the inclusion of organic monomers and polymers to provide a cell culture substrate tailored to specific cell-types. The cell culture system herein disclosed can be applied to both eukaryotic cells and prokaryotic cells to provide the means to produce cell cultures that mirror more closely in vivo conditions to provide a more reliable cell culture system that has applications, for example in tissue engineering, recombinant protein production and drug screening.

According to an aspect of the invention there is provided cell culture substrate comprising a plurality of sectioned microcellular polymeric material wherein said sections are 50-1000 microns and wherein the pore volume of the microcellular polymeric material is between 88% and 92%.

Pore volume is defined as the fraction of the total volume of the material that is comprised of pores, and is determined by the droplet fraction of the parent emulsion.

In a preferred embodiment of the invention said pore volume is about 90%.

We have determined that membranes of microcellular polymeric material with a pore volume of about 90% are a surprisingly effective substrate for cell growth. We have demonstrated that cell adherence, proliferation and function are significantly affected by the structure of the polymeric material. The cells adhere better to 90% porosity materials and proliferate well and show enhanced function over cells grown on polymeric materials with different porosities (for example, 95% pore volume). Furthermore, we have demonstrated that the proliferation and function of cells grown on 90% polymeric materials is significantly improved compared to the growth of cells on conventional 2-dimensional tissue culture plastic.

In a further preferred embodiment of the invention said substrate comprises a hydrophobic elastomer at a concentration of between 20 %(w/w) and 40% (w/w) of the total monomer content.

In a preferred embodiment said hydrophobic elastomer is provided at a concentration of between 25 %(w/w) and 35%(w/w). Preferably said concentration is selected from the group consisting of 26%(w/w); 27%(w/w); 28%(w/w); 29%(w/w); 30%(w/w); 31%(w/w); 32%(w/w); 33%(w/w); or 34 %(w/w).

In a preferred embodiment said hydrophobic elastomer is provided at a concentration of 30% (w/w).

In a preferred embodiment of the invention said elastomer is selected from the group consisting of: 2-ethylhexyl acrylate; n-butyl acrylate and n-hexyl acrylate.

In a preferred embodiment said elastomer is 2-ethylhexyl acrylate. Preferably said 2-ethylhexyl acrylate is provided at between 28 %(w/w) and 32% (w/w); preferably 2-ethylhexyl acrylate is provided at about 30% (w/w).

In a preferred embodiment said cell culture substrate comprises polyvinyl. Preferably said polyvinyl is polystyrene; preferably a polystyrene comprising a styrene monomer and divinylbenzene.

In a preferred embodiment of the invention said cell culture substrate comprises a surfactant.

In a preferred embodiment of the invention said surfactant is provided at a concentration of 20-30% (w/w) of the monomer phase of the emulsion; preferably 24-26 %(w/w) and most preferably around 25%(w/w).

In a preferred embodiment of the invention said cell culture substrate comprises a plurality of sectioned microcellular polymeric material wherein said sections are 50-1000 microns thick; preferably said sections are approximately 500-750 microns thick. More preferably still said sections are 100-200 microns thick.

In a preferred embodiment said cell culture substrate comprises a plurality of sectioned microcellular polymeric material wherein said sections are 50-250 microns thick; preferably said sections are approximately 150 microns thick.

In an alternative preferred embodiment said cell culture substrate comprises a plurality of sectioned microcellular polymeric material wherein said sections are 50-150 microns thick; preferably said sections are approximately 120 microns thick.

In a preferred embodiment said sectioned microcellular material is approximately 300 microns thick.

In a preferred embodiment said cell culture substrate comprises a further organic monomer.

In a preferred embodiment said organic monomer is selected from the group consisting of: N-butyl methacrylate, n-hexyl methacrylate, cyclohexyl acrylate, cyclohexyl methacrylate, phenyl acrylate, phenyl methacrylate, 3-vinylbenzyl chloride, 4-vinylbenzyl chloride, para-acetoxystyrene.

In a yet further preferred embodiment said cell culture substrate comprises a further organic polymer.

In a preferred embodiment said organic polymer is selected from the group consisting of: Poly(n-butyl methacrylate), poly(n-hexyl methacrylate), poly(cyclohexyl acrylate), poly(cyclohexyl methacrylate), poly(phenyl acrylate), poly(phenyl methacrylate), poly(3-vinylbenzyl chloride), poly(4-vinylbenzyl chloride), poly(para-acetoxystyrene).

In a preferred embodiment of the invention said cell culture substrate comprises a surface that has been modified by the provision of a coating that facilitates the attachment, proliferation and/or differentiation of cells attached to the surface.

In a preferred embodiment said modification is the provision of a proteinaceous coating.

In a preferred embodiment said proteinaceous coating comprises at least one molecule selected from the group consisting of: laminin, collagen, for example cell supports like Matrigel, fibronectin, non-collagen based peptide matrices.

An example of such a non-collagen based peptide matrix is PuraMatrix$^{tm}$.

In an alternative preferred embodiment said proteinaceous coating comprises a poly-amino acid coating.

Poly-amino acids have properties that mimic proteins and in particular proteins to which cells can attach and grow. Poly-amino acids can be homopolymers or heteropolymers. Examples of poly amino acids useful in cell culture include poly L ornthine and poly L lysine. Proteinaceous coatings are well known in the art. For example see Culture of Animal Cells, Ian Freshney, Wiley-Liss 1994.

In an alternative preferred embodiment the surface of said cell culture substrate is physically modified.

In a preferred embodiment said substrate comprises a surface that is modified by gas plasma treatment.

Gas plasma treatment of cell culture substrates is known in the art. The plasma treatment can be used to alter the physical properties of a cell culture surface. For example, ammonia and oxygen have been used as gas plasmas to improve cell attachment and proliferation on cell culture products. The process involves the excitation of gaseous products at low pressures and ambient temperatures by radio-frequency energy. The plasmas contain free electrons and other metastable particles which upon collision with polymeric surfaces can modify the surface by breaking chemical bonds. This creates free radicals which also modify the polymer surface.

According to a further aspect of the invention there is provided a cell culture vessel comprising a cell culture substrate according to the invention.

"Cell culture vessel" is defined as any means suitable to contain the above described cell culture substrate. Typically, an example of such a vessel is a petri dish; cell culture bottle or flask or multiwell culture dishes or well insert. Multiwell culture dishes are multiwell microtitre plates with formats such as 6, 12, 48, 96 and 384 wells which are typically used for compatibility with automated loading and robotic handling systems. Typically, high throughput screens use homogeneous mixtures of agents with an indicator compound that is either converted or modified resulting in the production of a signal. The signal is measured by suitable means (for example detection of fluorescence emission, optical density, or radioactivity) followed by integration of the signals from each well containing the cells, substrate/agent and indicator compound.

In a preferred embodiment said cell culture vessel comprising said cell culture substrate further comprises a cell and cell culture media.

In a preferred embodiment said cell is a eukaryotic cell; preferably said eukaryotic cell is selected from the group consisting of: a mammalian cell; a plant cell; a fungal cell; a slime mold.

In a preferred embodiment said mammalian cell is a primate cell; preferably said primate cell is a human cell.

In a preferred embodiment said mammalian cell is selected from the group consisting of: an epidermal keratinocyte; a fibroblast (e.g. dermal, corneal; intestinal mucosa, oral mucosa, bladder, urethral, prostate, liver) an epithelial cell (e.g. corneal, dermal, corneal; intestinal mucosa, oral mucosa, bladder, urethral, prostate, liver); a neuronal glial cell or neural cell; a hepatocyte or hepatocyte stellate cell; a mesenchymal cell; a muscle cell (cardiomyocyte, or myotube cell); a kidney cell; a blood cell (e.g. CD4+ lymphocyte, CD8+ lymphocyte; a pancreatic P cell; or an endothelial cell);

In a preferred embodiment said cell is a cell line derived from tumour tissue.

In an alternative preferred embodiment said mammalian cell is a stem cell except human embryonic stem cell.

In a preferred embodiment said stem cell is selected from the group consisting of: haemopoietic stem cell; neural stem cell; bone stem cell; muscle stem cell; mesenchymal stem cell; epithelial stem cell (derived from organs such as the skin, gastrointestinal mucosa, kidney, bladder, mammary glands, uterus, prostate and endocrine glands such as the pituitary); endodermal stem cell (derived from organs such as the liver, pancreas, lung and blood vessels); embryonic stem cell except human embryonic stem cell; embryonic germ cell except human embryonic ferm cell; embryonal carcinoma stem cell except human embryonal carcinoma stem cell.

In a preferred embodiment said embryonic stem cell/embryonic germ cell is a pluripotent cell and not a totipotent cell.

In an alternative preferred embodiment said cell is a prokaryotic cell; preferably a bacterial cell.

In a further preferred embodiment said cell or cell line is genetically modified.

In a preferred embodiment said cell culture vessel is a bioreactor; preferably said bioreactor is designed to scale-up the proliferation, differentiation and function of the said cell type.

According to an aspect of the invention there is provided a method for the culture of cells comprising the steps of:

i) providing a cell culture vessel comprising:

a) cells;
b) a cell culture substrate according to the invention;
c) cell culture medium sufficient to support the growth of said cells; and

ii) providing cell culture conditions which promote the proliferation and/or differentiation and/or function of said cells.

In a preferred method said cells are mammalian cells; preferably human cells.

In a preferred method said cells are hepatocytes.

In an alternative preferred embodiment said cells are prokaryotic cells; preferably bacterial cells.

If microorganisms are used in the cell culture method according to the invention, they are grown or cultured in the manner with which the skilled worker is familiar, depending on the host organism. As a rule, microorganisms are grown in a liquid medium comprising a carbon source, usually in the form of sugars, a nitrogen source, usually in the form of organic nitrogen sources such as yeast extract or salts such as ammonium sulfate, trace elements such as salts of iron, manganese and magnesium and, if appropriate, vitamins, at temperatures of between 0°C and 100°C, preferably between 10°C and 60°C, while gassing in oxygen.

The pH of the liquid medium can either be kept constant, that is to say regulated during the culturing period, or not. The cultures can be grown batchwise, semi-batchwise or continuously. Nutrients can be provided at the beginning of the fermentation or fed in semi-continuously or continuously. The products produced can be isolated from the organisms as described above by processes known to the skilled worker, for example by extraction, distillation, crystallization, if appropriate precipitation with salt, and/or chromatography. To this end, the organisms can advantageously be disrupted beforehand. In this process, the pH value is advantageously kept between pH 4 and 12, preferably between pH 6 and 9, especially preferably between pH 7 and 8.

As described above, these media which can be employed in accordance with the invention usually comprise one or more carbon sources, nitrogen sources, inorganic salts, vitamins and/or trace elements.

Preferred carbon sources are sugars, such as mono-, di- or polysaccharides. Examples of carbon sources are glucose, fructose, mannose, galactose, ribose, sorbose, ribulose, lactose, maltose, sucrose, raffinose, starch or cellulose. Sugars can also be added to the media via complex compounds such as molasses or other by-products from sugar refining. The addition of mixtures of a variety of carbon sources may also be advantageous. Other possible carbon sources are oils and fats such as, for example, soya oil, sunflower oil, peanut oil and/or coconut fat, fatty acids such as, for example, palmitic acid, stearic acid and/or linoleic acid, alcohols and/or polyalcohols such as, for example, glycerol, methanol and/or ethanol, and/or organic acids such as, for example, acetic acid and/or lactic acid.

Nitrogen sources are usually organic or inorganic nitrogen compounds or materials comprising these compounds. Examples of nitrogen sources comprise ammonia in liquid or gaseous form or ammonium salts such as ammonium sulfate, ammonium chloride, ammonium phosphate, ammonium carbonate or ammonium nitrate, nitrates, urea, amino acids or complex nitrogen sources such as comsteep liquor, soya meal, soya protein, yeast extract, meat extract and others. The nitrogen sources can be used individually or as a mixture.

Inorganic salt compounds which may be present in the media comprise the chloride, phosphorus and sulfate salts of calcium, magnesium, sodium, cobalt, molybdenum, potassium, manganese, zinc, copper and iron.

Inorganic sulfur-containing compounds such as, for example, sulfates, sulfites, dithionites, tetrathionates, thiosulfates, sulfides, or else organic sulfur compounds such as mercaptans and thiols may be used as sources of sulfur for the production of sulfur-containing fine chemicals, in particular of methionine.

Phosphoric acid, potassium dihydrogenphosphate or dipotassium hydrogenphosphate or the corresponding sodium-containing salts may be used as sources of phosphorus.

Chelating agents may be added to the medium in order to keep the metal ions in solution. Particularly suitable chelating agents comprise dihydroxyphenols such as catechol or protocatechuate and organic acids such as citric acid.

According to a further aspect of the invention there is provided a method to screen for an agent wherein said agent affects the proliferation, differentiation or function of a cell comprising the steps of:

i) providing a cell culture comprising at least one cell and a cell culture substrate according to the invention;
ii) adding at least one agent to be tested; and
iii) monitoring the activity of the agent with respect to the proliferation, differentiation or function of said cells.

In a preferred method said cell is a hepatocyte.

In a preferred method said screening method includes the steps of: collating the activity data in (iii) above; converting the collated data into a data analysable form; and optionally providing an output for the analysed data.

A number of methods are known which image and extract information concerning the spatial and temporal changes occurring in cells expressing, for example fluorescent proteins and other markers of gene expression, (see Taylor et al Am. Scientist 80: 322-335, 1992). Moreover, US5, 989,835 and US09/031,271 disclose optical systems for determining the distribution or activity of fluorescent reporter molecules in cells for screening large numbers of agents for biological activity. The systems disclosed in the above patents also describe a computerised method for processing, storing and displaying the data generated.

The screening of large numbers of agents requires preparing arrays of cells for the handling of cells and the administration of agents. Assay devices, for example, include standard multiwell microtitre plates with formats such as 6, 12, 48, 96 and 384 wells which are typically used for compatibility with automated loading and robotic handling systems. Typically, high throughput screens use homogeneous mixtures of agents with an indicator compound which is either converted or modified resulting in the production of a signal. The signal is measured by suitable means (for example detection of fluorescence emission, optical density, or radioactivity) followed by integration of the signals from each well containing the cells, agent and indicator compound.

The term "agent" includes any small molecule, antibody, polypeptide, peptide, aptamer, double stranded or small inhibitory RNA. These can be an agonist or an antagonist.

Small molecule antagonists include chemotherapeutic agents useful in the treatment of diseases such as cancer.

Antibodies or immunoglobulins (Ig) are a class of structurally related proteins consisting of two pairs of polypeptide chains, one pair of light (L) (low molecular weight) chain (κ or λ), and one pair of heavy (H) chains (γ, α, μ, δ and ε), all four linked together by disulphide bonds. Both H and L chains have regions that contribute to the binding of antigen and that are highly variable from one Ig molecule to another. In addition, H and L chains contain regions that are non-variable or constant. The L chains consist of two domains. The carboxy-terminal domain is essentially identical among L chains of a given type and is referred to as the "constant" (C) region. The amino terminal domain varies from L chain to L chain and contributes to the binding site of the antibody. Because of its variability, it is referred to as the "variable" (V) region. The variable region contains complementarity determining regions or CDR's which form an antigen binding pocket. The binding pockets comprise H and L variable regions which contribute to antigen recognition. It is possible to create single variable regions, so called single chain antibody variable region fragments (scFv's). If a hybridoma exists for a specific monoclonal antibody it is well within the knowledge of the skilled person to isolate scFv's from mRNA extracted from said hybridoma via RT PCR. Alternatively, phage display screening can be undertaken to identify clones expressing scFv's. Alternatively said fragments are "domain antibody fragments". Domain antibodies are the smallest binding part of an antibody (approximately 13kDa). Examples of this technology is disclosed in US6, 248, 516, US6,291, 158, US6,127, 197 and EP0368684.

Aptamers are small, usually stabilised, nucleic acid molecules which comprise a binding domain for a target molecule. A screening method to identify aptamers is described in US 5,270,163. Aptamers are typically oligonucleotides which may be single stranded oligodeoxynucleotides, oligoribonucleotides, or modified oligodeoxynucleotide or oligoribonucleotides.

A more recent technique to specifically ablate gene function is through the introduction of double stranded RNA, also referred to as small inhibitory or interfering RNA (siRNA), into a cell which results in the destruction of mRNA complementary to the sequence included in the siRNA molecule. The siRNA molecule comprises two complementary strands of RNA (a sense strand and an antisense strand) annealed to each other to form a double stranded RNA molecule. The siRNA molecule is typically derived from exons of the gene which is to be ablated. The mechanism of RNA interference is being elucidated. Many organisms respond to the presence of double stranded RNA by activating a cascade that leads to the formation of siRNA. The presence of double stranded RNA activates a protein complex comprising RNase III which processes the double stranded RNA into smaller fragments (siRNAs, approximately 21-29 nucleotides in length) which become part of a ribonucleoprotein complex. The siRNA acts as a guide for the RNase complex to cleave mRNA complementary to the antisense strand of the siRNA thereby resulting in destruction of the mRNA. An agent based on a siRNA would have value in determining the function of a specific gene in cell proliferation and/or differentiation.

According to a further aspect of the invention there is provided a method for the identification of genes associated with cell differentiation comprising the steps of:

 i) providing a cell culture comprising at least one cell and a cell culture substrate according to the invention;

 ii) extracting nucleic acid from cells in said cell culture;

 iii) contacting said extracted nucleic acid with a nucleic acid array; and

 iv) detecting a signal which indicates the binding of said nucleic acid to a binding partner on said nucleic acid array.

In a preferred method said cell is a hepatocyte.

Preferably said method includes the additional steps of:

i) collating the signal(s) generated by the binding of said nucleic acid to said binding partner;

ii) converting the collated signal(s) into a data analysable form; and optionally;

iii) providing an output for the analysed data.

Methods used in the identification of cell differentiation markers and/or markers of cell transformation include immunogenic based techniques (e.g. using the cells as complex immunogens to develop antisera to for example cell surface markers and the like) nucleic acid based techniques (e.g. differential screening using cDNA from normal and transformed cells). Also, it has been known for many years that tumour cells produce a number of tumour cell specific antigens, some of which are presented at the tumour cell surface. These are generally referred to as tumour rejection antigens and are derived from larger polypeptides referred to as tumour rejection antigen precursors. Tumour rejection antigens are presented via HLA's to the immune system. The immune system recognises these molecules as foreign and naturally selects and destroys cells expressing these antigens. If a transformed cell escapes detection and becomes established a tumour develops. Vaccines have been developed based on dominant tumour rejection antigens to provide individuals with a preformed defence to the establishment of a tumour. The method according to the invention provides a means to identify tumour rejection antigens and precursors which will have utility with respect to the vaccine development to provoke the patients own immune system to deter the establishment of tumours.

According to a yet further aspect of the invention there is provided an *in vitro* method to analyse the development of cancerous cells from normal cells comprising

i) forming a preparation comprising a cell culture substrate according to the invention including cells;

ii) adding at least one agent capable of inducing cell transformation; and

iii) monitoring the effect, or not, of said agent on the transformation of said cells.

In a preferred method said cells are hepatocytes.

It is well known in the art that there are agents capable of transforming a normal cell into a transformed cell with many of the features of cancerous cells. These include, by example only, viruses, DNA intercalating agents, oncogenes and telomerase genes.

As used herein, the term "cancer" or "cancerous" refers to cells having the capacity for autonomous growth, i.e., an abnormal state or condition characterized by rapidly proliferating cell growth. The term is meant to include all types of cancerous growths or oncogenic processes, metastatic tissues or malignantly transformed cells, tissues, or organs, irrespective of histopathologic type or stage of invasiveness. The term "cancer" includes malignancies of the various organ systems, such as those affecting, for example, lung, breast, thyroid, lymphoid, gastrointestinal, and genitourinary tract, as well as adenocarcinomas which include malignancies such as most colon cancers, renal-cell carcinoma, prostate cancer and/or testicular tumours, non-small cell carcinoma of the lung, cancer of the small intestine and cancer of the esophagus. The term "carcinoma" is art recognized and refers to malignancies of epithelial or endocrine tissues including respiratory system carcinomas, gastrointestinal system carcinomas, genitourinary system carcinomas, testicular carcinomas, breast carcinomas, prostatic carcinomas, endocrine system carcinomas, and melanomas. Exemplary carcinomas include those forming from tissue of the cervix, lung, prostate, breast, head and neck, colon and ovary. The term "carcinoma" also includes carcinosarcomas, e.g., which include malignant tumours composed of carcinomatous and sarcomatous tissues. An "adenocarcinoma" refers to a carcinoma derived from glandular tissue or in which the tumor cells form recognizable glandular structures. The term "sarcoma" is art recognized and refers to malignant tumours of mesenchymal derivation.

According to a further aspect of the invention there is provided a process for the formation of a high internal phase polymer having a pore volume of between 88% and 92% comprising the steps of:

i) forming a preparation comprising an high internal phase emulsion comprising a hydrophobic elastomer at a concentration of between 20%(w/w) and 40% (w/w) and a surfactant provided at a concentration of between 20-30% (w/w);

ii) forming a preparation comprising a catalyst;

iii) combining the preparations in (i) and (ii);

iv) incubating the combined preparation to allow formation of a high internal phase emulsion polymer; and

v) sectioning said polymer into parts that are 50-1000 microns thick.

In a preferred method said hydrophobic elastomer is provided at a concentration of between 25 %(w/w) and 35%(w/w); preferably said hydrophobic elastomer is provided at a concentration of about 30% (w/w).

In a preferred method said elastomer is selected from the group consisting of: 2-ethylhexyl acrylate; n-butyl acrylate and n-hexyl acrylate.

In a preferred method the temperature of the preparation in ii) is heated to a temperature of between 50°C and 80°C.

In a further preferred method said preparation in ii) is heated to 50°C or 60°C or 80°C.

In a further preferred method said preparation in i) comprises a styrene monomer.

In a further preferred method said preparation in i) comprises divinyl benzene.

In a yet further preferred method said preparation in i) comprises a surfactant that is provided at a concentration of 24-26 %(w/w) and most preferably around 25%(w/w).

In a preferred method the preparation in i) comprises 60%(w/w) styrene; 30% (w/w) 2-ethylhexyl acrylate; 10% (w/w) divinylbenzene and 25% surfactant.

In a preferred method said high internal phase emulsion polymer in step iv) is sectioned; preferably said polymer is sectioned into a thin membrane or layer.

In a preferred method said polymer is engineered into a thin membrane or layer of approximately 50-150 microns thick; preferably said membranes are approximately 120 microns thick.

According to a further aspect there is provided the use of a substrate comprising a high internal phase emulsion polymer to determine the liver toxicity of an agent.

In a preferred embodiment said agent is a chemotherapeutic agent.

In an alternative preferred embodiment said agent is a viral gene therapy vector.

According to a further aspect of the invention there is provided a method to test the liver toxicity of an agent comprising the steps of:

> i) providing a cell culture comprising at least one hepatocyte cell and a cell culture substrate according to any of claims 1-5;
> ii) adding at least one agent to be tested; and
> iii) monitoring the activity of the agent with respect to the proliferation, differentiation or function of said hepatocyte cells as a measure of toxicity of the agent.

In a preferred method said agent is a chemotherapeutic agent.

In an alternative preferred method said agent is a viral gene therapy vector.

According to a further aspect of the invention there is provided a method for the growth and differentiation of a keratinocyte and/or keratinocyte precursor stem cell comprising:

> i) forming a preparation comprising a cell culture substrate according to the invention, fibroblast feeder cells and cell culture medium;
> ii) culturing said feeder cells to provide a cell culture substrate that is substantially coated with said feeder cells;
> iii) contacting said coated substrate with keratinocytes and/or keratinocyte precursor stem cells; and
> iv) culturing the combined cell preparation under conditions conducive to the growth and differentiation of said keratinocytes and/or keratinocyte precursor stem cells.

In a preferred method said fibroblast feeder cells are dermal fibroblasts.

In an alternative preferred method said fibroblast feeder cells are selected from the group consisting of: corneal fibroblasts, intestinal mucosa fibroblasts, oral mucosa fibroblasts, urethral fibroblasts, or bladder fibroblasts.

In a further preferred method said keratinocytes are epidermal keratinocytes.

In a preferred method said fibroblasts are human fibroblasts.

In a further preferred method said keratinocytes are human keratinocytes.

In a preferred method said preparation further comprises collagen.

In a preferred method collagen is type 1 collagen.

In a further preferred method said collagen is provided as a gel.

In an alternative preferred method said collagen is provided in a solution.

In a further preferred method at least said keratinocytes are displaced to contact air thereby inducing keratinocyte stratification.

In a preferred method of the invention there is provided a method to test an agent comprising:

> i) forming a preparation according to the invention which includes an agent to be tested;
> ii) monitoring the effect of said agent on keratinocyte cell growth and/or differentiation when compared to a control preparation that does not include said agent

According to a further aspect of the invention there is provided an apparatus for the culture of cells comprising a cell culture substrate according to any of claims 1-31, a cell culture vessel and an insert adapted to co-operate with said cell culture vessel and contain said cell culture substrate and said cells.

In a preferred embodiment said cell culture substrate comprises fibroblasts and keratinocytes.

According to a yet further aspect of the invention there is provided the use of a substrate according to the invention for the preparation of differentiated skin composite. Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

An embodiment of the invention will now be described by example only and with reference to the following figures:

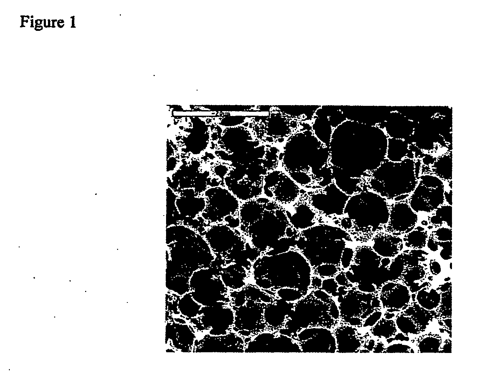

Figure 1 is a scanning electron micrograph (SEM) image of a typical PolyHIPE material. The spherical cavities in Figure 1 are voids, the holes joining adjacent voids are called interconnects. Scale bar = 20□m;

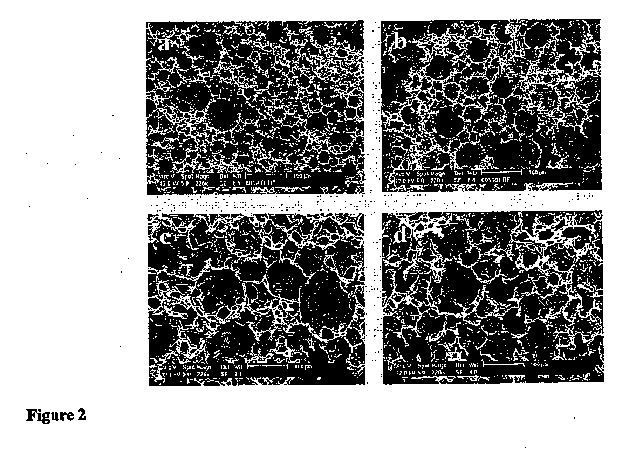

Figure 2 shows SEM images of PolyHIPE materials prepared with different aqueous phase temperatures: (a) room temperature; (b) 50°C; (c) 60 °C; (d) 80°C. Scale bar = 100 □m;

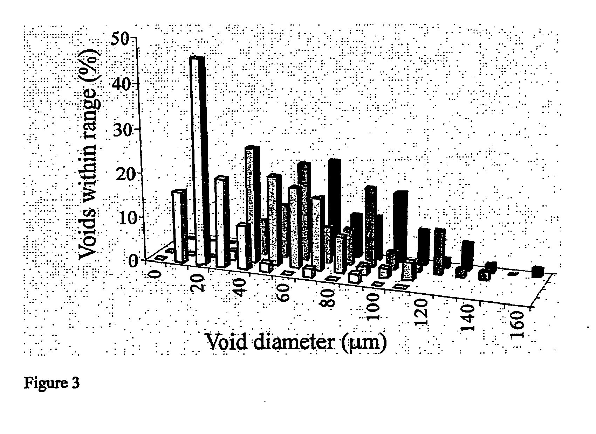

Figure 3 illustrates the influence of aqueous phase temperature on void diameter distribution. From front to back: room temperature, 50 °C, 60 °C, 80°C;

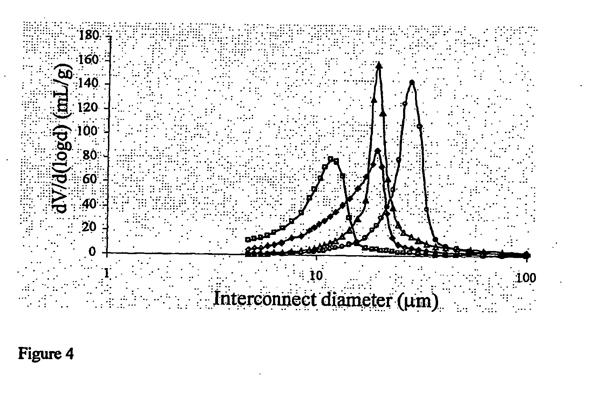

Figure 4 illustrates interconnect size distribution of PolyHIPE materials produced using different aqueous phase temperatures: room temperature (□); 50 °C (◊); 60 °C (Δ); 80 °C(○);

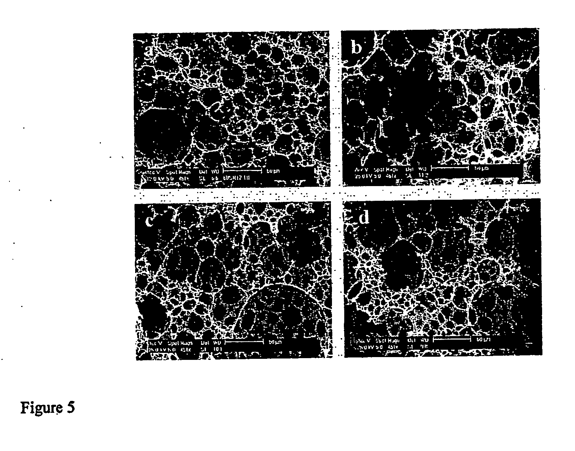

Figure 5 shows the influence of aqueous phase additives on PolyHIPE morphology: (a) no additive; (b) 1.5 % (w/v) PEG ($M_n$ = 300); (c) 4% (v/v) methanol; (d) 1.5% (v/v) THF. Scale bar = 50 □m.

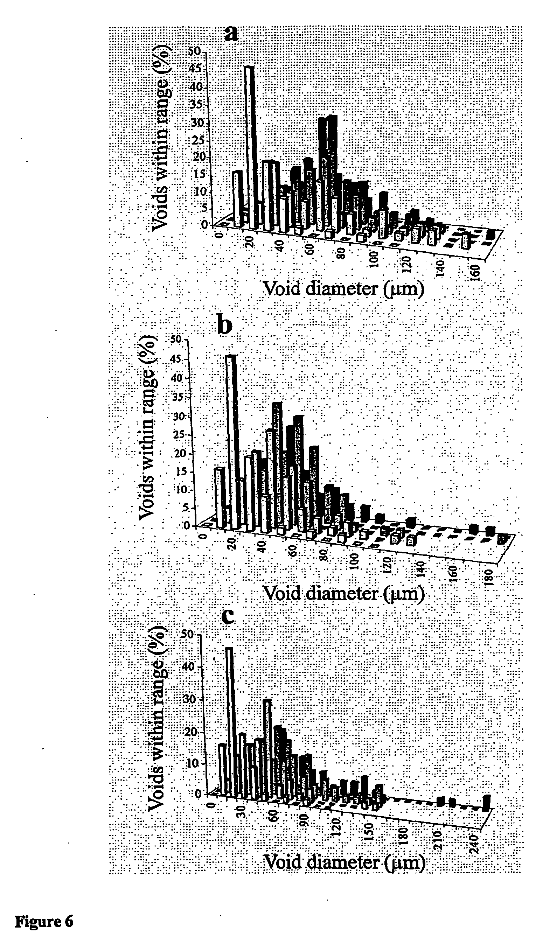

Figure 6 illustrates void diameter distribution plots for PolyHIPE materials prepared with aqueous phase additives: (a) PEG (from front to back: no PEG, 0.2%, 0.4%, 0.8%, 1.5%); (b) methanol (from front to back: no methanol, 1%, 2%, 3%, 4%); (c) THF (from front to back: no THF, 0.4%, 0.8%, 1%, 1.5%). PEG $M_n$ = 300; all percentages expressed as v/v, except PEG which is w/v. In each case the aqueous phase was kept at room temperature during emulsion preparation;

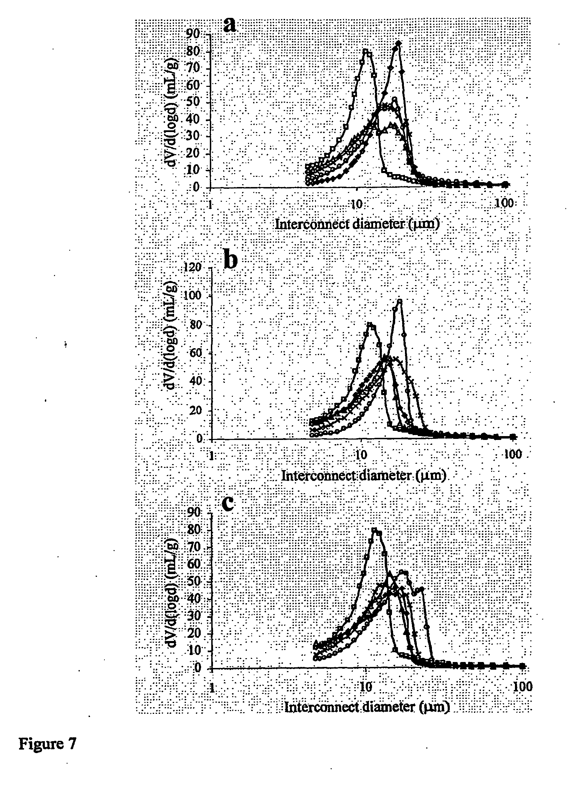

Figure 7 illustrates interconnect size distribution of PolyHIPE materials produced using different aqueous phase additives: (a) PEG (□ no PEG, Δ 0.2%, × 0.4%, ○ 0.8%, ◊ 1.5%); (b) methanol (□ no methanol, Δ 1%, ◊ 2%, ○ 3%, × 4%); (c) THF (□ no THF, ◊ 0.4%, Δ 0.8%, × 1%, ○ 1.5%). PEG $M_n$ = 300; all percentages expressed as v/v, except PEG which is w/v. In each case the aqueous phase was kept at room temperature during emulsion preparation;

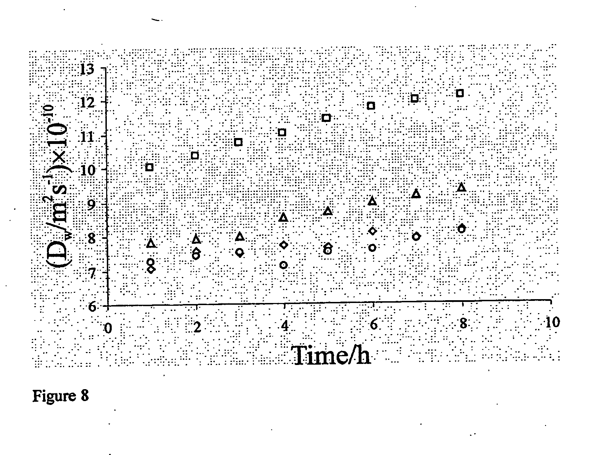

Figure 8 illustrates self diffusion coefficient of water in HIPEs prepared with different aqueous phase additives (Ono additive; □ 1.5 % THF; Δ 1.5 % PEG; ○ 2 % methanol). PEG $M_n$ = 300; all percentages expressed as v/v, except PEG which is w/v. In each case the aqueous phase was kept at room temperature during emulsion preparation;

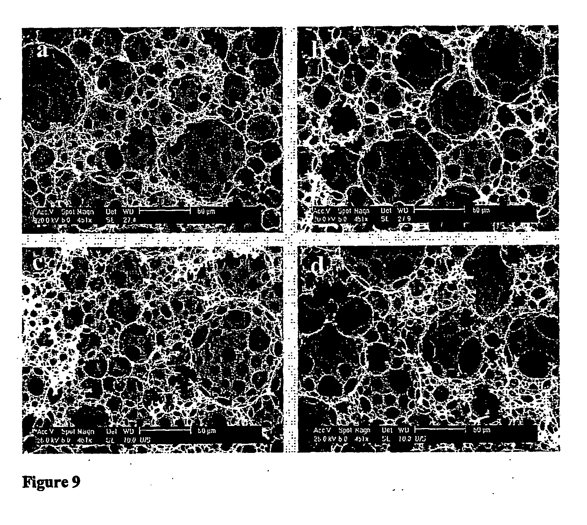

Figure 9 shows SEM images of PolyHIPE materials prepared with different surfactant concentrations ($C_S$) in the presence of aqueous phase additives: 1.5 % THF, $C_s$ = 20 % (a); 1.5 % THF, $G_s$ = 30 % (b); 4 % methanol, $C_s$ = 20 % (c); 4 % methanol, $C_s$ = 30 % (d). Scale bar = 50 □m. PEG $M_n$ = 300; all percentages expressed as v/v, except PEG which is w/v. In each case the aqueous phase was kept at room temperature during emulsion preparation.

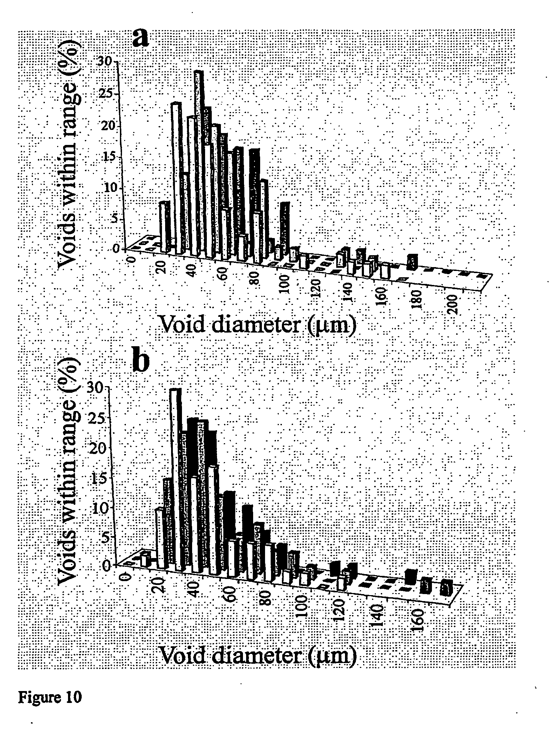

Figure 10 illustrates void diameter distribution plots for PolyHIPE materials prepared with different surfactant concentrations in the presence of additives: 1.5 % THF (a); 4 % methanol (b). From front to back: $C_s$ = 30, 25 and 20 % (w/w). PEG $M_n$ = 300; all percentages expressed as v/v, except PEG which is w/v. In each case the aqueous phase was kept at room temperature during emulsion preparation.

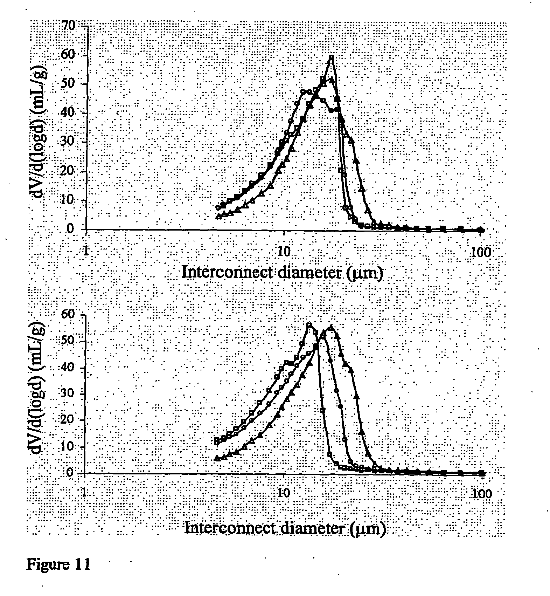

Figure 11 illustrates interconnect size distribution of PolyHIPE materials produced using different surfactant concentrations ($C_s$) in the presence of aqueous phase additives: (a) 1.5 vol. % THF; (b) 4 vol. % methanol (□: $C_s$ = 20%; △: $C_s$ = 25%; ○: $C_s$ = 30%; all percentages expressed as v/v). In each case the aqueous phase was kept at room temperature during emulsion preparation.

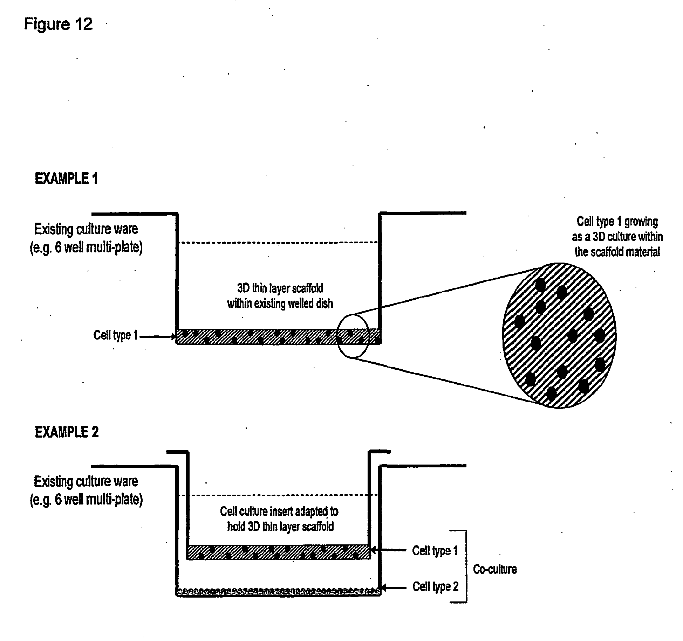

Figure 12 illustrates an example application of styrene-based polyHIPE scaffolds as thin membranes adapted for use in existing cell culture vessels such as a multi-welled plate or well insert.

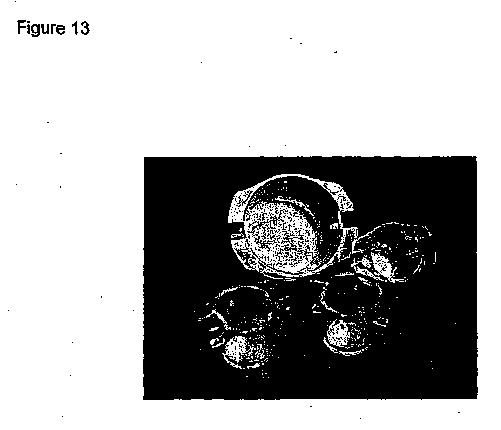

Figure 13 shows a photograph of prototype well inserts carrying the 90% pore volume polystyrene scaffold at 120 microns thick. These examples are of inserts designed to fit into 6-welled (large insert) and 12-welled (small inserts) culture plates.

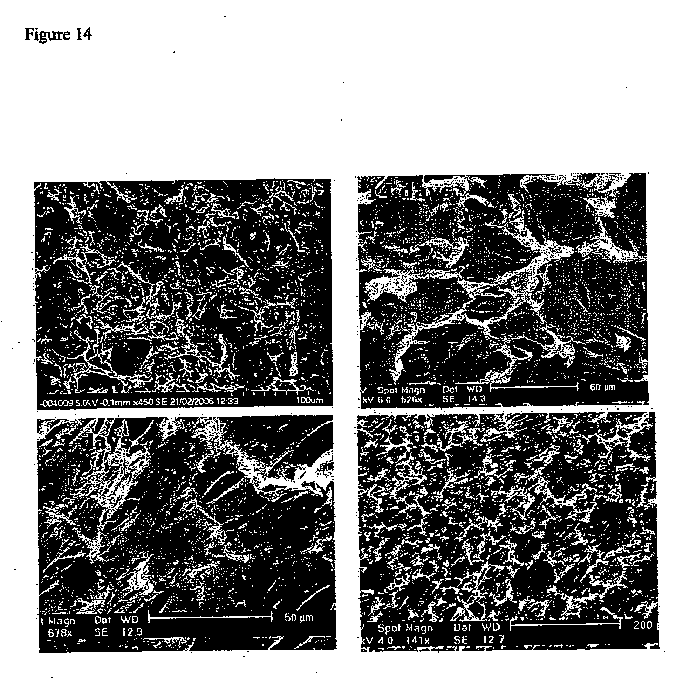

Figure 14 is a SEM showing MG63 osteoblasts cultured on 90% pore volume polystyrene scaffolds for 7-28 days in vitro. These materials have been adapted for use in existing cell culture plastic-ware as illustrated in Figure 12.

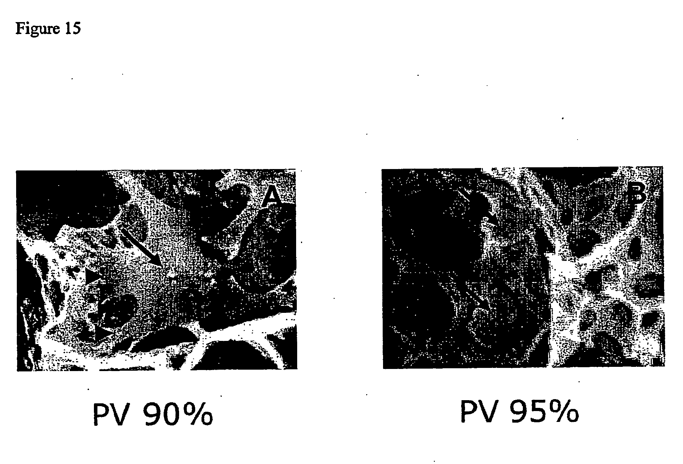

Figure 15 demonstrates that the preparation and structural characteristics of the polymer affect the growth of cells within the scaffold (example: 90% versus 95% pore volume). This example shows how cell morphology is affected. Scanning electron micrographs of MG63 osteoblasts cultured on polystyrene scaffolds for 7 days in vitro. These materials were produced using pore volumes (PV) of 90% and 95%. (A) Osteoblasts (arrow) grown on 90% polymers spread out and exhibited numerous lamellipodia (arrowheads) enhancing interactions with neighbouring cells. (B) However, cells (arrows) grown 95% polymers maintained a rounded appearance and produced fewer if any lamellipodia. (Images are of similar magnification).

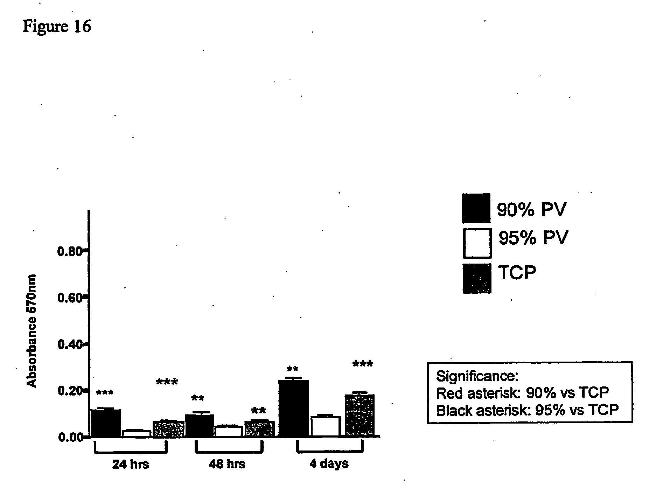

Figure 16 illustrates how the structure of the growth substrate can influence cell behaviour. The data show significant differences in the proliferation rate of cells grown on various types of substrate. Specifically note the comparison between polymers of 90% and 95% pore volumes. This demonstrates the importance of tailoring these scaffolds for cell growth. The figure shows data from a MTT cell proliferation assay of cultured MG63 osteoblasts grown on either 90% or 95% pore volume (PV) polystyrene scaffolds, or flat, conventional tissue culture plastic (TCP). Cells were seeded at $1 \times 10^6$ cells per well. Bars represent the mean $\pm$ SEM, n=3. Note that cell proliferation is significantly greater on 90% scaffolds compared to TCP and 95% PV materials. These data also show that cells proliferate the least on scaffolds made with 95% PV.

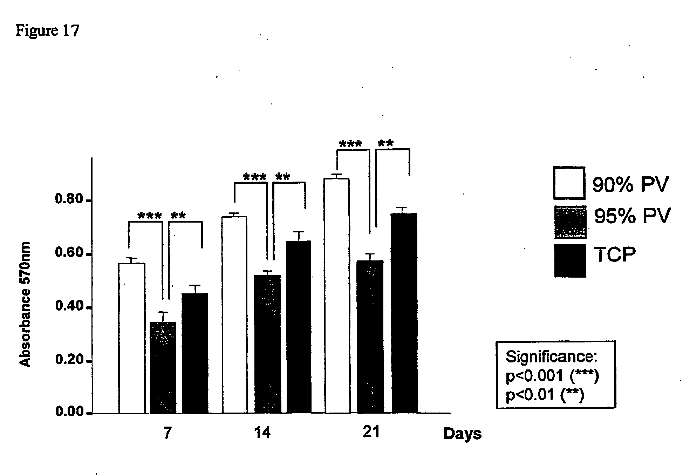

Figure 17 illustrates how the structure of the growth substrate can influence cell behaviour. The data show significant differences in the proliferation rate of cells grown on various types of substrate. Specifically note the comparison between polymers of 90% and 95% pore volumes. This demonstrates the importance of tailoring these scaffolds for cell growth. The figure shows data from a MTT cell proliferation assay of cultured bone marrow derived mesenchymal stem cells (MSCs) grown on either 90% or 95% pore volume (PV) polystyrene scaffolds, or flat conventional tissue culture plastic (TCP). Cells were seeded at $1 \times 10^6$ cells per well. Bars represent the mean $\pm$ SEM, n=3. Again, these data show that cell proliferation is significantly greater on 90% scaffolds compared to TCP and 95% PV materials. In addition, cells proliferate the least on scaffolds made with 95% PV.

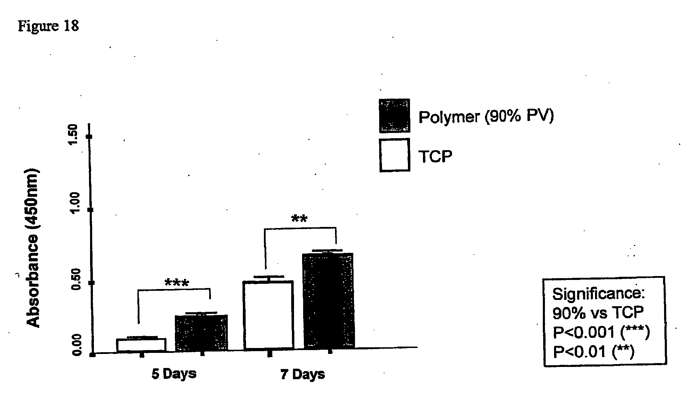

Figure 18 shows significant differences in the function of cells grown on 3-dimensional 90% pore volume polystyrene scaffolds compared to their growth on 2-dimensional conventional tissue culture plastic. Assay measuring the levels of alkaline phosphatase in MG63 osteoblasts cultured on 90% pore volume (PV) scaffolds compared to flat, conventional tissue culture plastic (TCP) for 5 and 7 days. Cells were seeded at $1 \times 10^6$ cells per well. Values have been normalised to account for any differences in cell number. Bars represent the mean $\pm$ SEM, n=3. Note that alkaline phosphatase levels are significantly higher in cultures of osteoblasts grown on 3-dimensional polystyrene compared to flat polystyrene surfaces. These data show enhanced activity of these cells when grown on the 3-dimensional scaffold compared to conventional 2-dimensional culture plastic.

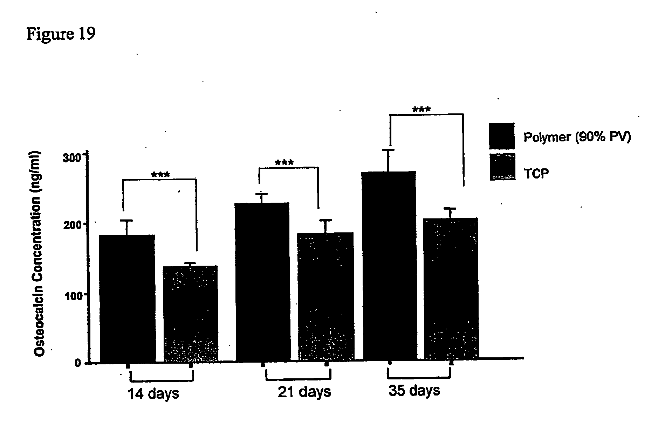

Figure 19 shows significant differences in the function of cells grown on 3-dimensional 90% pore volume polystyrene scaffolds compared to their growth on 2-dimensional conventional tissue culture plastic. Assay measuring the levels of osteocalcin in bone marrow derived MSCs induced to form bone nodules in response to dexamethasone. Cells were cultured on either 90% pore volume (PV) polystyrene scaffolds or flat, conventional tissue culture plastic (TCP) for 14 to 35 days. Cells were seeded at $1 \times 10^6$ cells per well. Values have been normalised to account far any differences in cell number. Bars represent the mean $\pm$ SEM, n=3. Note that osteocalcin concentrations are significantly higher in cultures of differentiating cells grown on 3-dimensional polystyrene compared to flat polystyrene surfaces. These data again show enhanced activity of these cells when grown on the 3-dimensional scaffold compared to conventional 2-dimensional culture plastic.

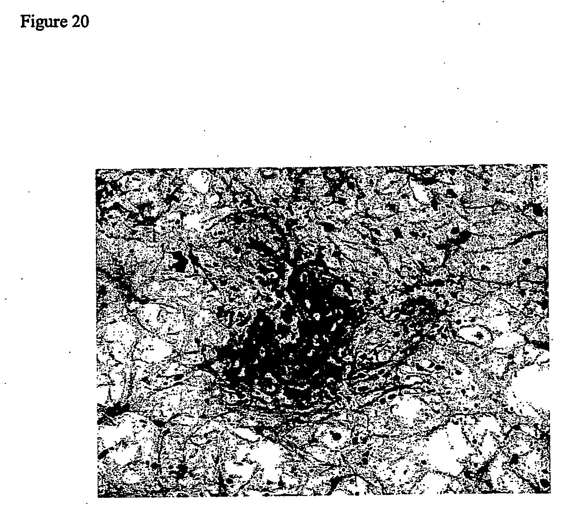

Figure 20 is a photomicrograph of Von Kossa staining showing the formation of a centrally located bone nodule. The bone nodule was derived from mesenchymal stems induced to differentiate with dexamethasone when grown within a 90% pore volume polystyrene scaffold. Cells are counterstained with Mayor's Haematoxylin.

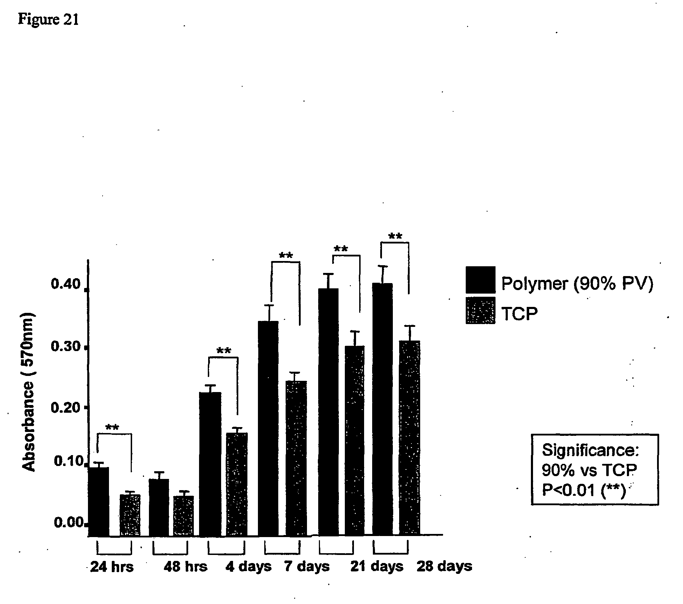

Figure 21 illustrates how the structure of the growth substrate can influence cell behaviour. The data exemplify the advantage of growing cells within a 90% pore volume polystyrene scaffold compared to conventional tissue culture plastic. The figure shows data from a MTT cell proliferation assay of cultured HEP G2 hepatocytes grown on either 90% pore volume (PV) polystyrene scaffold or flat, conventional tissue culture plastic (TCP). Cells were seeded at $1x10^6$ cells/well. Bars represent the mean $\pm$ SEM, n=3. Note that cell proliferation is significantly greater on 90% scaffolds compared to 2-dimensional TCP.

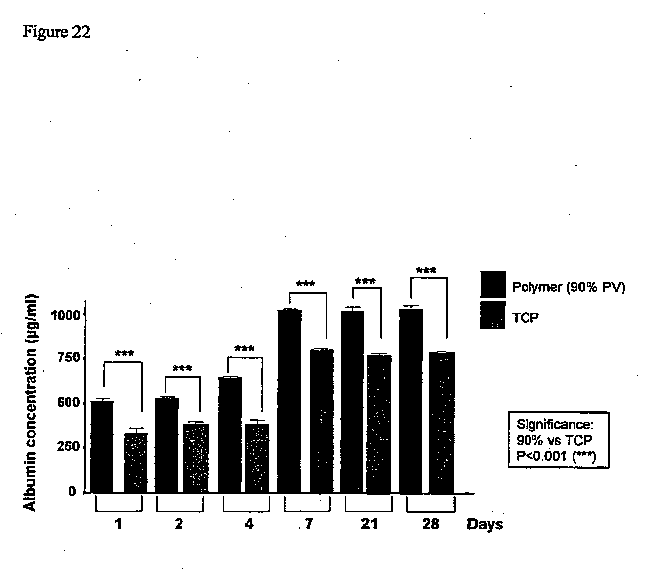

Figure 22 illustrates how the structure of the growth substrate can influence cell function. The data exemplify the advantage of growing cells within a 90% pore volume polystyrene scaffold compared to conventional tissue culture plastic. Assay measuring the levels of albumin production from HEP G2 hepatocytes cultured on either 90% pore volume (PV) polystyrene scaffolds or flat, conventional tissue culture plastic (TCP) for 1 to 28 days. Cells were seeded at $1x10^6$ cells per well. Values have been normalised to account for any differences in cell number. Bars represent the mean $\pm$ SEM, n=3. Note that albumin concentrations are significantly higher in cultures of differentiating cells grown on 3-dimensional polystyrene compared to flat polystyrene surfaces. These data again suggest enhanced activity of these cells when grown on the 3-dimensional scaffold compared to those cultured on the flat surface of conventional plastic-ware.

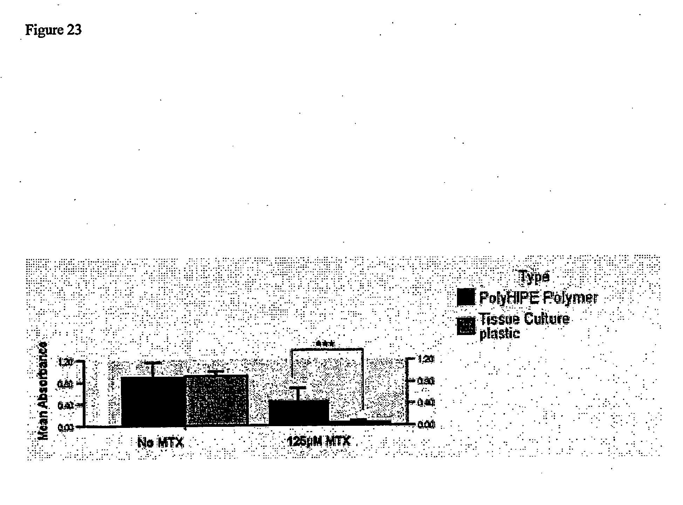

Figure 23 illustrates how the structure of the growth substrate can influence cell function, in this case, the enhanced tolerance of cells to cytotoxic challenge. The data exemplify the advantage of growing cells within a 90% pore volume polystyrene scaffold compared to conventional tissue culture plastic. The figure shows data from a MTT cell proliferation assay of cultured HEP G2 hepatocytes grown on either 90% pore volume (PV) polystyrene scaffold or flat, conventional tissue culture plastic (TCP) for 3 days in the presence (125 microM) or absence of the cytotoxin methotrexate (DNA synthesis inhibitor). Cells were seeded at $1x10^6$ cells/well. Bars represent the mean $\pm$ SEM, n=3. Note that cell proliferation is significantly greater on 90% scaffolds compared to 2-dimensional TCP. These data suggest that cells grown on scaffolds are more tolerant to this cytotoxin under these growth conditions.

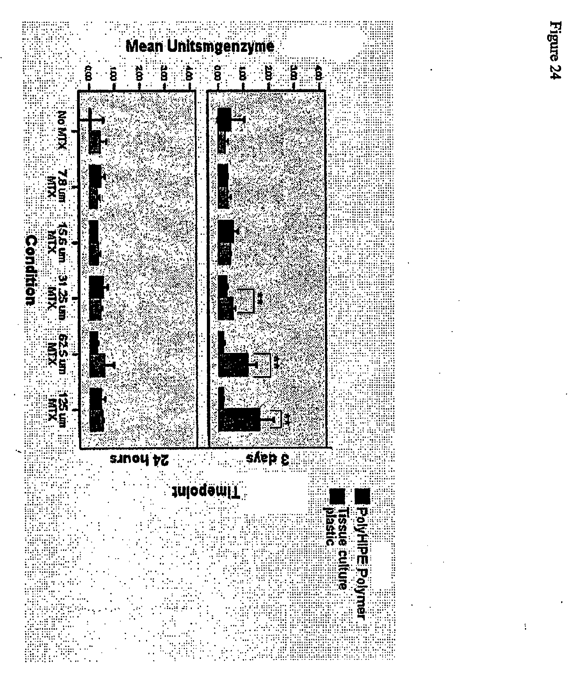

Figure 24 illustrates how the structure of the growth substrate can influence cell function and further exemplify the differences in growing cells within a 90% pore volume polystyrene scaffold compared to conventional tissue culture plastic. Assay measuring the levels of transglutaminase in cultures of HEP G2 hepatocytes grown on either 90% pore volume (PV) polystyrene scaffolds or flat, conventional tissue culture plastic (TCP) for 1 to 3 days. Cells were seeded at $1x10^6$ cells per well. Values have been normalised to account for any differences in cell number. Bars represent the mean $\pm$ SEM, n=3. Transglutaminase is a protein cross-linking enzyme known to be expressed by hepatocytes and is induced as hepatocytes enter apoptosis. Note that levels of transglutaminase are significantly higher in hepatocyte cultures grown on flat polystyrene surfaces compared to 3-dimensional polystyrene when challenged with increasing concentrations of the cytotoxin methotrexate. These data further suggest that cells on scaffolds are more tolerant to these levels of cytotoxic challenge which may be consequence of their growth under less stressful conditions unlike those experienced by cells grown as 2-dimensional monolayers;

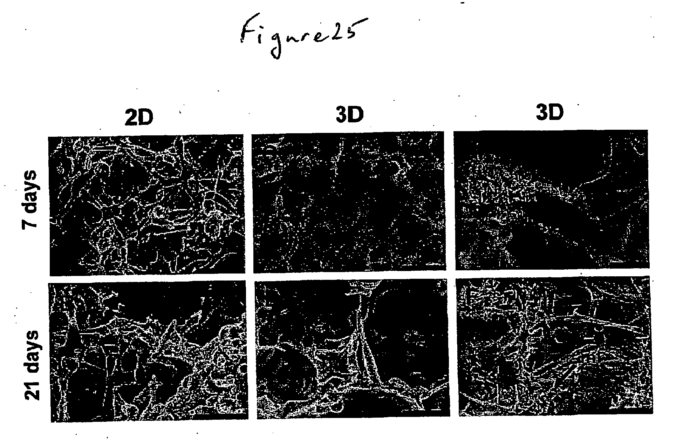

Figure 25: Scanning electron micrographs showing HepG2 hepatocytes cultured on 2-D (A,B) and 3-D (C-F) polystyrene substrates for either 7 days (A,C,E) or 21 days (B,D,F). Hepatocytes grown on 2-D substrates appeared significantly more heterogeneous in structure (A,B), compared to cells grown on 3-D surfaces (C). A decreased seeding density enabled visualisation of individual cells grown on 3-D scaffolds (sc) (D). HepG2 cells developed complex 3-D shapes and interactions with neighbouring cells (D). Higher magnification images revealed the expression of large numbers of micro-villi (mv) on the surface of cells (E,F). There were consistently greater numbers of micro-villi on cells grown in 3-D (C-F) compared to cells grown on 2-D surfaces (A,B). Scale bars: A-D 25$\mu$m; E,F 5$\mu$m.

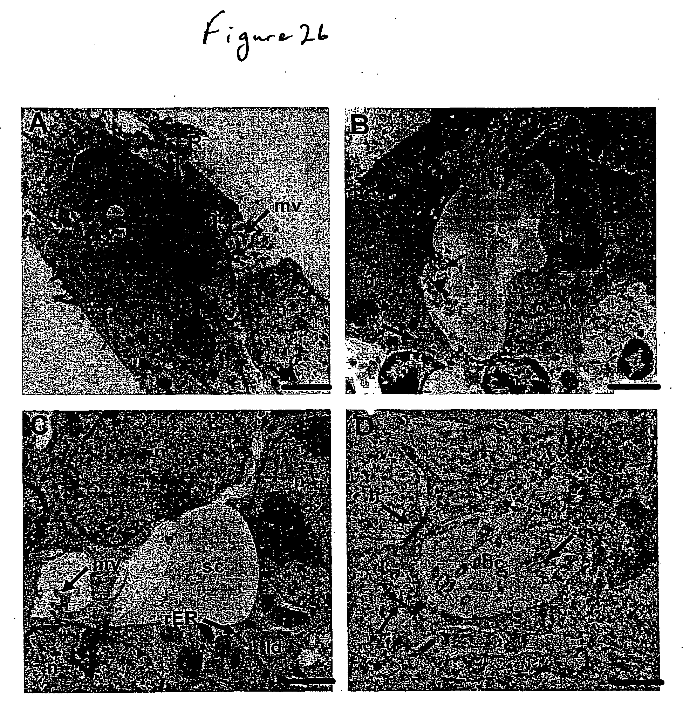

Figure 26: Transmission electron micrographs showing the ultra-structural features of HepG2 cells cultured on either 2-D or 3-D surfaces for 21 days. (A) HepG2 cells cultured on 2-D plastic exhibited numerous clearly identifiable organelles, including nuclei (n), mitochondria (mt), rough endoplasmic reticulum (rER), micro-villi (mv), and lipid droplets (ld). (B,C) HepG2 cells cultured on polystyrene scaffolds (sc) grow in close association with the polymer, completely surrounding struts of the material as shown. Imaging showed that cells grown in 3-D also displayed an array of cellular organelles such as nuclei (n), mitochondria (mt), rough endoplasmic reticulum (rER), micro-villi (mv), lipid droplets (ld) and peroxisomal clusters (pc). (D) High magnification micrograph showing the formation of tight junction (tj) complexes between adjacent cells. The void formed in between cells closely resembles a bile canaliculus (be) into which project micro-villi (mv). Scale bars: A,B 2$\mu$m; C 1$\mu$m; D 500nm.

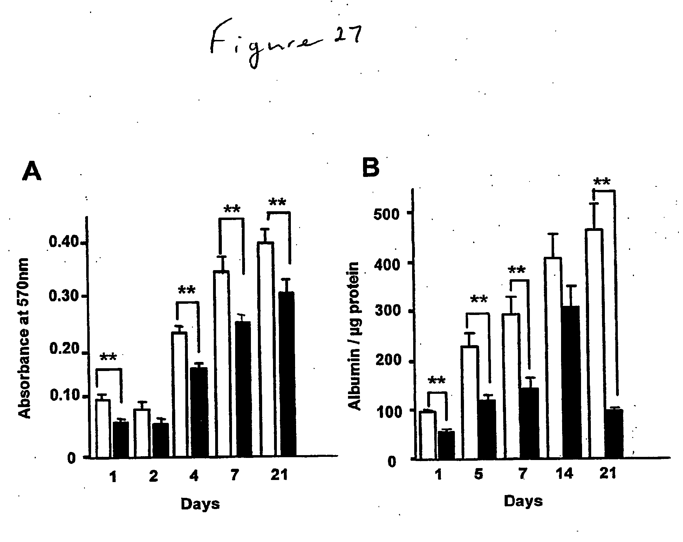

Figure 27: Performance of HepG2 cells cultured on 2-D (solid bars) and 3-D (open bars) polystyrene substrates cultured for 21 days. (A) Assessment of cell viability using MTT assay. (B) Production of albumin secreted by HepG2 cells into the culture medium. Albumin secretion was normalised to the total amount of protein per well. For both experiments, cells were seeded at 1x10$^6$cells/well. Data represent the mean $\pm$SEM for three independent repeats. Significance is denoted by **$p$<0.01 using the Mann Whitney U test

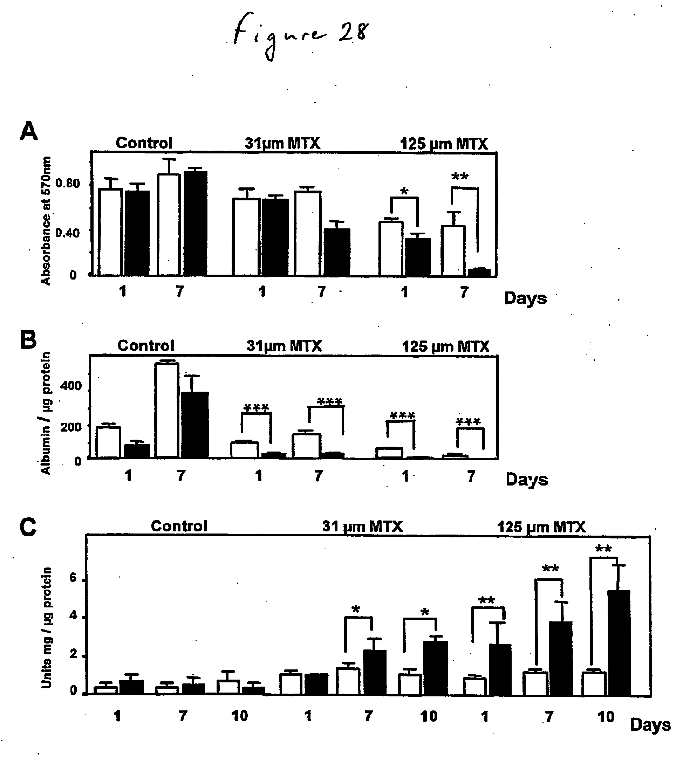

Figure 28: Performance of HepG2 cells cultured on 2-D (solid bars) and 3-D (open bars) substrates when challenged by the cytotoxin, methotrexate (MTX). Data show cells were treated either with vehicle alone (control), or 31$\mu$M MTX, or 125$\mu$M MTX for up to 10 days. (A) Measure of cell viability using MTT assay. (B) Determination HepG2 cell metabolic activity by measurement of albumin secretion into the culture medium. Albumin levels were normalised to the total amount of protein per well. (C) Assessment of cell damage as determined by transglutaminase activity. Enzyme levels were normalised to the total amount of protein per well. For each experiment (A-C), cells were seeded at 1$\times$10$^6$cells/well. Data represent the mean $\pm$SEM for three independent repeats. Significance is denoted by *$p$<0.05, **$p$<0.01 and ***$p$<0.001 using the Mann Whitney U test.

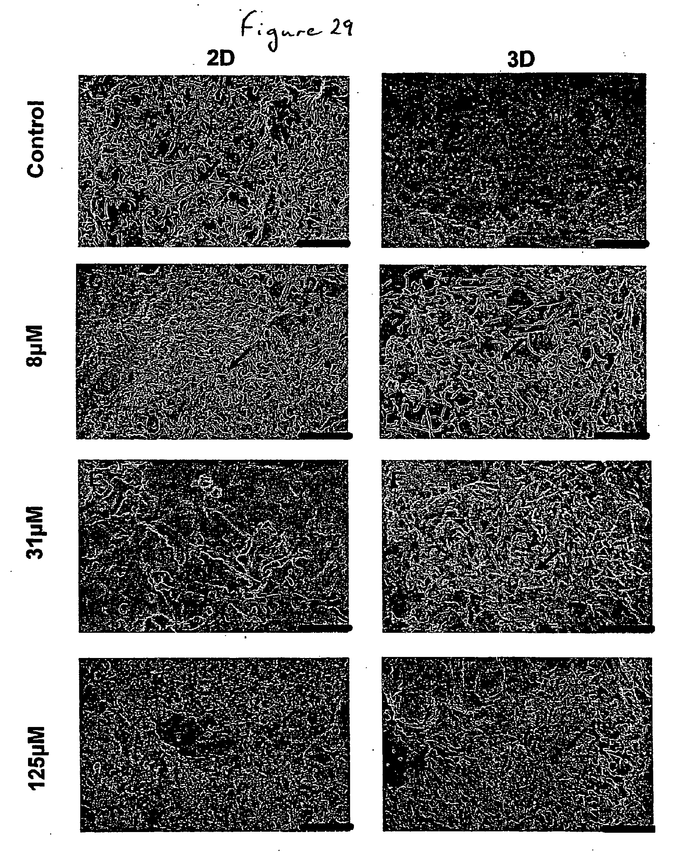

Figure 29: Scanning electron micrographs showing the effect of methotrexate (MTX on the surface structure of HepG2 cells. Image panels show HepG2 cells were cultured on 2-D (A,C,E,G) and 3-D (B,D,F,H) substrates, treated with either vehicle (control, no MTX, (A,B)), 8$\mu$M (C,D), 31$\mu$M (E,F), or 125$\mu$M (G,H) MTX. Note that micro-villi (mv) on the cell surface are clearly visible in both control cultures (A,B) and cells exposed to low concentrations of MTX (C,D) when grown on either 2-D (A,C) or 3-D (B,D) substrates. At higher concentrations of the cytotoxin, cells grown on 2-D substrates possessed very few micro-villi (E) and the cell surface showed evidence of breaking up at the maximum levels of MTX tested (F). In contrast, HepG2 cells grown in 3-D and exposed to increasing levels of MTX remained intact and exhibited large numbers of micro-villi (F,H). Scale bars: A-H 2$\mu$m.

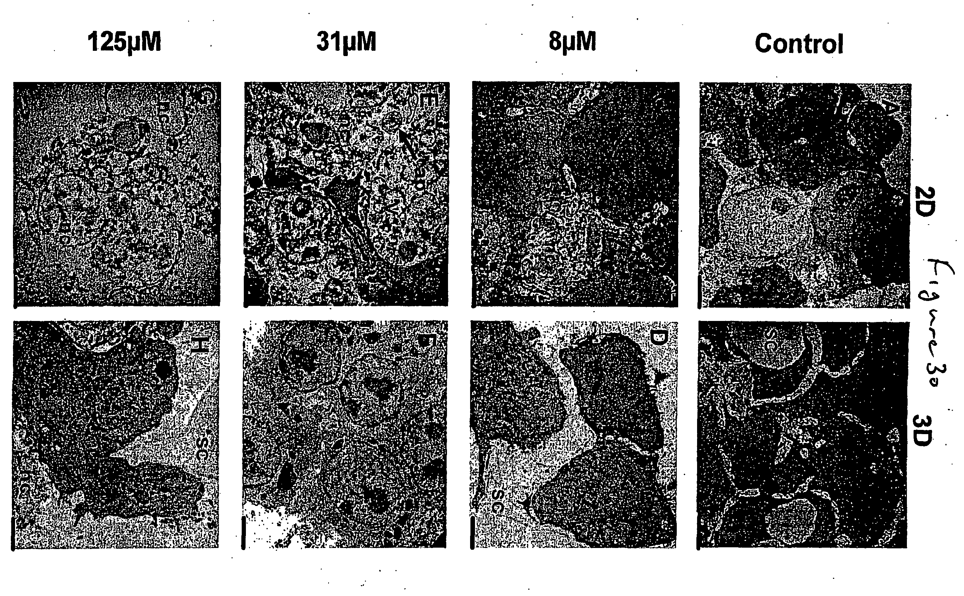

Figure 30: The effect of methotrexate (MTX) on the ultra-structure of HepG2 cells. Micrographs show cultured cells on 2-D (A,C,E,G) and 3-D (B,D,F,H) substrates, treated with either vehicle (control, no MTX, (A,B)), 8$\mu$M (C,D), 31$\mu$M (E,F), or 125$\mu$M (G,H) MTX. Images of control cultures show the normal structure of cells corresponding to the growth substrate (A,B). The majority of cells grown on flat tissue culture plastic and exposed to 8$\mu$M MTX possessed near normal cellular architecture although a few necrotic cells were identified (C, nc). Increasing concentrations of MTX resulted in the destruction of the vast majority of cells grown on 2-D substrates (E,G). Nuclear membranes had disintegrated and organelles normally found in healthy cells could be identified. There was an increased presence of large vacuolar spaces (v) and membranous bodies known as autophagolysosomes (ap) (E). In contrast, HepG2 cells grown on 3-D scaffolds maintained their structure and only a small number of necrotic cells (nc) were identified in cultures exposed to the 125$\mu$M MTX (H). Scale bars A-H 2$\mu$m.

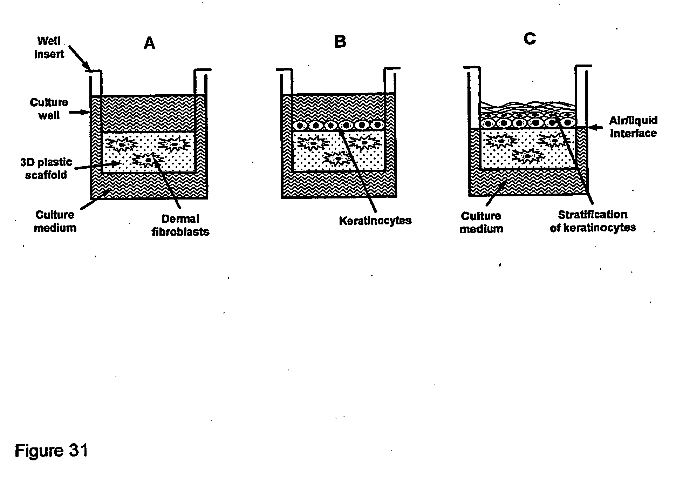

Figure 31: Example configuration for organotypic coculture of mammalian skin epithelial cells. (A) Well insert with 3D porous polystyrene scaffold attached to base, located in culture well of multi-welled dish (e.g. 6-well plate). Dermal fibroblasts grow within 3D polystyrene scaffold in the presence or absence of collagen gel. (B) Keratinocytes (e.g. HaCaT cells) seeded onto surface of dermal fibroblast culture. (C) Exposure of epidermal keratinocytes to air induces cell stratification achieved in this case by lowering level of culture medium. Cells grown on the 3D scaffolds are readily transferable between different cell culture vessels allowing improved handling by the user.

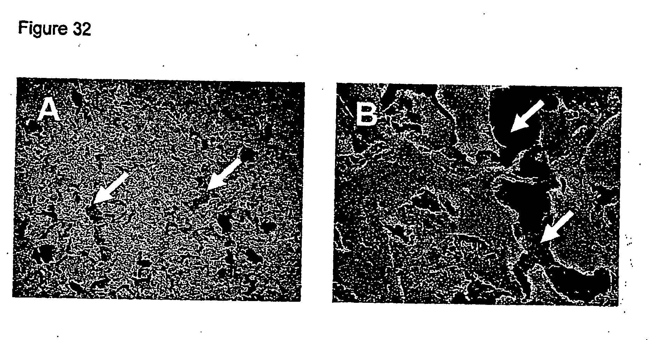

Figure 32: Scanning electron micrographs of dermal fibroblasts grown on 3D polystyrene scaffolds shown at low (A) and high (B) magnifications. Arrows indicate exposure of the scaffold beneath layer of cells. Structural support of cells improves handling of cultures for routine manipulations by users.

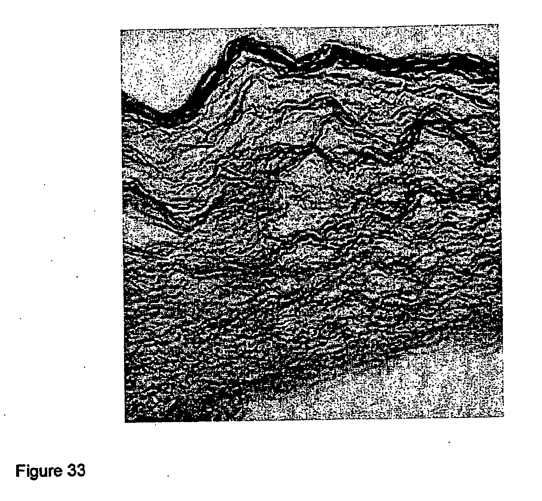

Figure 33: Stratification of human keratinocytes (HaCaT cells) in organotypic cocultures with fibroblasts grown in 3D. Preparation prepared for histological analysis, sectioned, and epithelial cells stained with Hematoxylin and Eosin.

Table 1 Morphological Parameters of PolyHIPEs Prepared with Different Aqueous Phase Temperatures and Water-miscible Additives;

Table 2 Average Void and Interconnect Diameters of PolyHIPEs Prepared with Aqueous Phase Additives, and Water Self-diffusion Coefficient Values in the Parent HIPEs[a]; and

Table 3 Influence of Surfactant Concentration on Morphology of PolyHIPEs Prepared with Aqueous Phase Additives.

**Materials and Methods for the Production of Growth substrate for Routine Use in Cell Culture**

**Materials** Divinylbenzene (Aldrich; 80 vol % divinylbenzene, the remainder being m- and p-ethylstyrene), 2-ethylhexyl acrylate (Aldrich; 99 %) and styrene (Aldrich; 99 %) were passed through a column of basic activated alumina (Aldrich; Brockmann 1) to remove any inhibitor (4-tert-butylcatechol for styrene and divinylbenzene and hydroquinone or monomethyl ether hydroquinone for 2-ethyhexyl acrylate). Potassium persulfate (Aldrich), sorbitan monooleate (SPAN 80, Aldrich), poly(ethylene glycol) (Aldrich, $M_n$ = 300) and calcium chloride dihydrate (Aldrich) were used as supplied.

**Preparation of PolyHIPE Polymers and Fabrication into Thin Membranes for Cell Culture**

PolyHIPE foams were prepared using the polymerisation of a HIPE.

- The oil phase contained 60% styrene, 30% 2-ethylhexyl acrylate, 10% divinylbenzene and 25% surfactant (sorbitan monooleate) (all % are w/w).

- The aqueous phase contained 1% potassium persulphate in de-ionised $H_2O$.

**Method**

1. In brief, the oil phase was placed in a 3-necked 250mL round- bottomed flask, fitted with an overhead stirrer (glass rod fitted with a D-shaped PTFE paddle), a 100 mL pressure equalising dropping funnel (inserted into a side-neck) and a rubber septum. The mixture was purged with nitrogen gas for 15 min.

2. The aqueous phase was heated up to a temperature of 80°C using a stirrer hotplate and then added to the oil phase over a period of 2 minutes at a constant rate. The emulsion was then mixed for a further minute.

3. The emulsion was then removed and cast in a 50ml polypropylene tube and left to cure at 60°C overnight.

4. The polymer was then removed from the tube after 24 hrs and washed extensively in a soxhlet with water and isopropyl alcohol for 24 hrs each.

**Production of Thin membranes**

The polymers were engineered into 120 micron thick membranes. This can be achieved using a microtome or vibrotome should thicker sections (up to 1mm) be required. Membranes of polymeric material were then sterilized using absolute ethanol, hydrated through a series of graded ethanol solutions and subsequently washed ($\times$3) with sterile phosphate buffered saline (PBS) prior to use. Membranes can be mounted directly into the bottom of existing cell culture plastic-ware (e.g. 6-welled plate) or adhered to a cell culture well insert (see Figures 12 and 13).

**Scanning Electron Microscopy**

The morphologies of the materials were investigated using a FEI XL30 ESEM operating at between 20-25 kV. Fractured segments were mounted on carbon fibre pads and attached to aluminium stubs and were gold coated using an Edwards Pirani 501 sputter coater. The calculation of average void size was performed using the image analysis software Image J (NIH image). Average diameters measured in this way are underestimates of the real values. Therefore it is necessary to introduce a statistical correction[1]. This is achieved by evaluating the average of the ratio R/r, where R is the equatorial value of void diameter and r is the diameter value measured from the micrograph. The statistical factor is calculated from eq. (1).

$$h^2 = R^2 - r^2 \qquad (1)$$

The probability that the sectioning takes place at any distance (h) from the centre is the same for all values of h, so the average probability value of h is R/2. Replacing this value in eq. (1) gives R/r = 2/(3^{1/2}). Multiplication of the observed average value of the void diameter allows a more accurate value to be obtained.

### Mercury Intrusion Porosimetry

Mercury intrusion porosimetry analysis was performed using a Micromeritics AutoPore III 9420. Intrusion and extrusion mercury contact angles of 130° were used. Penetrometers with a stem volume of 1.836 mL and a bulb volume of 5 mL were used. The intrusion volume always comprised between 45 and 80 % of the stem volume. Intrusion pressures for the PolyHIPEs never exceeded 200 psi.

### $^1$H NMR Diffusion Experiments

The self diffusion coefficient of water ($D_w$) was measured using a 500 MHz Varian Unity Inova 500 narrow bore spectrometer equipped with a Performa II gradient pulse amplifier and an actively shielded 5 mm indirect direction probe. Automated z gradient shimming based on deuterium spin echoes was used. The temperature used for all measurements was 25 +/- 0.1 °C. Water diffusion coefficients were measured using a pulse sequence incorporating pulsed-field gradients such as the bipolar pulse pair stimulated echo (BPPSTE) pulse sequence. Diffusion coefficients are obtained from BPPTSE spectra by monitoring signal attenuation as a function of the applied magnetic field gradient amplitude and fitting eq. (2) to the experimental results.

$$I = I_0 \exp\left[ -D(\gamma \delta G)^2 \left( \Delta - (\delta/2) - (\tau/3) \right) \right] \qquad (2)$$

In eq. (2), I is the resonance intensity measured for a given gradient amplitude, G, $I_0$ is the intensity in the absence of the gradient pulse, $\gamma$ is the gyromagnetic ratio, $\delta$ is the duration of the bipolar gradient pulse pair, $\Delta$ is the diffusion delay time and $\tau$ is a short gradient recovery delay time during which relaxation and spin-spin coupling evolution are not significant.

### *Hepatocyte cell culture*

The human hepatic carcinoma cell line, HepG2, was obtained from the American Type Culture Collection (ATCC). HepG2 cells were cultured at 37°C in 5% $CO_2$ in growth medium (Dulbecco's modified Eagle medium (D-MEM, Gibco/BRL) supplemented with 10% (v/v) foetal calf serum (FBS, Gibco/BRL), 100$\mu$g.mL$^{-1}$ penicillin and 10$\mu$g.mL$^{-1}$ streptomycin (Gibco/BRL)). Cells were passaged every 5-7 days. Confluent cultures of cells were washed with PBS, detached using trypsin/EDTA solution and cell number determined using a hemocytometer. Suspensions of HepG2 cells were then seeded at equal densities either directly into wells of a standard 6-welled plate (Nunc) or into modified well-inserts mounted with the polymer and located in wells of a 6-well plate. Cultures were maintained in growth medium which was changed every 3-4 days or as required.

### *Determination of viable cell number*

The number of viable cells was determined using a commercially available colorimetric assay (Promega) based on Mosmann's original method for measuring cell activity involving the conversion of a tetrazolium salt into a blue formazan product detectable by a spectrophotometer (570nm) [32]. The assay was performed according to the manufacturer's instructions on HepG2 cells cultured on 2-D and 3-D substrates for various periods under alternative growth conditions.

### *Methotrexate (MTX) toxicity studies*

Cells were seeded on 2-D and 3-D surfaces in triplicate and left to settle and adhere for 24 hours. The medium was then changed and replaced with medium containing different concentrations of MTX (no MTX (vehicle alone, control), 8$\mu$M, 31$\mu$M, and 125$\mu$M). Cells were subsequently incubated for 1, 3, 7 or 10 days, after which cultures were sampled and assayed for cell number/viability and levels of albumin and transglutaminase were determined.

*Hepatocyte metabolic activity*

The production of albumin is often used as an indicator of hepatocyte metabolic activity. Levels of albumin were determined using a commercially available kit (Bioassay systems) based on an established method that utilizes bromocresol green which forms a coloured complex specifically with albumin that is detectable at 620nm. Known quantities of human albumin were used to establish the standard curve. Specific levels of albumin secretion were normalised to total protein levels (as determined by a standard Bradford assay).

*Preparation of samples for scanning electron microscopy (SEM) and transmission electron microscopy (TEM)*

In preparation for SEM, cells grown on 2-D or 3-D substrates were fixed in 2% paraformaldehyde and 2.5% glutaraldehyde in Sorenson's phosphate buffer for 1 hour at room temperature. Samples were then rinsed in 0.1M phosphate buffer and immersed in 1% $OsO_4$ (aq.) solution for 1 hour, then dehydrated in 50%, 70%, 95% and 100% ethanol for 5 minutes, four times for each respective ethanol change. Samples of fixed 2-D and 3-D cultures were then cut into smaller pieces (approximately $25mm^2$), mounted on specimen holders and dried from $CO_2$ at 38°C at 1200 psi. The samples were then sputter coated with a 7nm layer of chromium and examined using a Hitachi S5200 SEM.
For TEM analysis, cells grown on 3-D substrates were fixed and treated as described above for SEM. However, subsequent to dehydration and being cut into small pieces, samples were embedded in resin (Araldite CY212, Agar Scientific) for 1 hour at 37°C and then placed into pyramidal moulds at 60°C overnight. For the preparation of cells grown on 2-D surfaces, cultures were fixed in 2% paraformaldehyde and 2.5% glutaraldehyde in Sorenson's phosphate buffer for 1 hour at room temperature. Cells were then scrapped from tissue culture plastic and pelleted at 15,000rpm for 10min. Pelleted cells were subsequently rinsed in 0.1M phosphate buffer and immersed in 1% $OsO_4$ (aq.) solution for 1 hour, then dehydrated in 50%, 70%, 95% and 100% ethanol for 5min, for times for each respective ethanol change. The dehydrated cell pellets were then soaked in resin (Araldite CY212) for 60min at 37°C. When set, ultra thin sections of the resin embedded material were produced and subsequently imaged by TEM (Hitachi H7600).

*Enzymatic assay of transglutaminase*

Tissue transglutaminase is a cross-linking enzyme which has recently been suggested to play a role in the formation of fibrotic lesions in experimental settings. The leakage of this enzyme is often used as a marker for *in vitro* toxicity testing and its presence indicates damage to cell membranes. Several *in vivo* and *in vitro* experimental model systems show a direct relationship between the expression and activity of tissue transglutaminase, suppression of cell growth and programmed cell death [33-35]. The level of transglutaminase was analysed by means of a quantitative enzymatic assay (Sigma, UK) as previously described [36].

*Statistical analysis*

Experiments were performed as at least three independent replicates. Data were analysed for statistical significance using the Mann Whitney U test (at the 5% level of significance or greater).

## EXAMPLE 1

### Effect of Aqueous Phase Temperature

Increasing the temperature of the aqueous phase was found to cause a striking increase in both the average interconnect and void diameter of the PolyHIPE material (Figure 2). Average void diameters were calculated from a set of 50 voids, the diamaters of which were determined by image analysis of the SEM micrographs. The calculated average void diameter values (<D>) show a steady increase with increasing aqueous phase temperature (Table 1), which is most likely due to the decreasing HIPE stability as the aqueous phase temperature is increased. Increasing the temperature of the aqueous phase, and therefore thermal agitation of the water droplets, will increase the frequency of contact and will result in a higher probability of droplet coalescence[2]. Lissant also reported that, as the emulsion is subjected to heating, the surfactant in the interfacial film separating the droplets becomes more soluble in the bulk liquid phase and therefore migrates from the interface[3]. This will raise the interfacial tension and thus promote droplet coalescence. It is also noticeable that, as the aqueous phase temperature is increased, the viscosity of the HIPE decreases suggesting that the droplets have a higher mobility. This also helps to promote coalescence.
It has been described in the literature[4] that the droplet size distribution for an emulsion undergoing coalescence contains the presence of a tail extending towards larger droplet sizes with the maximum staying relatively unchanged. The void size distribution plot (Figure 3) shows the distribution tailing towards larger void sizes. The tail increases with temperature and an increased broadening of the distribution is also observed. This therefore reinforces the opinion that coalescence is the main mechanism of emulsion instability as the aqueous phase temperature is increased.

The differential plot of intrusion versus interconnect diameter (Figure 4) shows an increase in interconnect size as the aqueous phase temperature is increased. The plot also shows that, as the aqueous phase temperature is increased, a material with a narrower distribution at higher interconnect diameters and a tail extending in the lower interconnect diameter range exists. This suggests that, for each emulsion, a limiting interconnect diameter exists. This is in contrast to the void size distribution, where a broader distribution is obtained as the temperature is increased. The ratio of the average interconnect (<d>) and void diameters (<D>) provides a measure of the degree of interconnection. The values for the materials prepared in this study are shown in Table 1. As the temperature is increased, the degree of interconnection (<d>/<D>) of the PolyHIPE material decreases. This suggests that, as the aqueous phase temperature is increased, the emulsion stability decreases.

### EXAMPLE 2

### Effect of additives

Emulsion partial destabilisation can be induced by the presence of organic additives in the aqueous phase. These additives should be partially soluble in both the continuous and the internal phase of the emulsion, which can thus enhance diffusion of water molecules from droplet to droplet and promote Ostwald ripening. Lissant reported[3] that addition of cosolvents, such as acetone or methanol, can disrupt the interfacial film due to their solubility in both phases. These additives may dilute the interfacial layer and cause some of the surfactant to migrate into the bulk phase, therefore promoting coalescence of the emulsion droplets.

Poly(ethylene glycol) of $M_n$=300 ($PEG_{300}$), methanol and tetrahydrofuran (THF) were chosen as water-miscible organic species, with a view to selecting species with a range of molar masses and of different polarities. Each component was added to HIPEs in increasing quantities until phase separation occurred. It was found that the emulsion could accept much higher quantities of methanol than either THF or PEG, and this is particularly apparent if one considers the molar quantities of each (0.1 mol methanol, 0.02 mol THF and 0.005 mol PEG). This is due to the greater partitioning of methanol into the aqueous phase, at least in comparison to THF. The octanol/water partition coefficient value (log $P_{ow}$) for THF is 0.45, whereas that of methanol is - 0.77. No log $P_{ow}$ value could be found for PEG. The SEM images (Figure 5) suggest that each additive produces an increase in the average void and interconnect size. Image analysis of the SEM micrographs produces the void size distributions, which are shown in Figure 6. Figure 6a shows that the addition of PEG produces a material with a wider distribution of void sizes than the PolyHIPE material with no additive present, with a tail extending towards larger void sizes. This pattern is similar to that obtained for methanol (Figure 6b) and THF (Figure 6c), however with methanol the effect on the distribution is less than that for THF or PEG. The materials prepared with THF or PEG contain a wider range of void sizes than the materials prepared with methanol. PEG and THF also produced PolyHIPE materials with higher average void size values (see Table 1).

The interconnect distribution curves are similar in nature for all additives used (Figure 7). As the concentration of additive is increased, there is a tendency towards materials with a higher average interconnect diameter and a narrower size distribution. The exception to this trend is when THF is used as the additive. In this case a broad distribution is still obtained at high THF concentration. When PEG was used as the additive, the average interconnect diameter values increased steadily with PEG concentration in the aqueous phase (Table 1). This effect was not as pronounced for THF or methanol; for these additives, much higher concentrations were needed to produce a significant change in the interconnect diameter. In the case of THF, this was unexpected as it had the most significant effect on the average void diameter. The degree of interconnection (<d>/<D>) decreases following initial addition of the organic component, which is brought about by the large increase in void diameter compared to interconnect diameter. After the initial addition of PEG or methanol, the degree of interconnection increases steadily with the concentration in the aqueous phase. However, in the case of THF, the degree of interconnection continued to decrease with increasing concentration. This is because THF has no significant effect on the interconnect size until the concentration reaches 1.5 %.

In previous work[5] the increase in void size of PolyHIPE materials on addition of a co-solvent or additive has been ascribed solely to Ostwald ripening. However, it is possible that organic additives influence other processes that lead to emulsion destabilisation. As discussed previously, the addition of a co-solvent can disrupt the interfacial layer by causing surfactant to migrate into the bulk phase, causing the emulsion droplets to become more prone to coalescence. It is possible that the rate of both coalescence and Ostwald ripening are enhanced by the addition of co-solvents to the system, and that one process may dominate depending on the exact system (emulsion type, surfactant type, etc.).

In order to probe the influence of Ostwald ripening, the self diffusion coefficient of water, $D_w$, was monitored in the presence of each additive over a period of 8 hours (Table 2). When methanol is present there is no significant difference in the diffusion coefficient compared to the emulsion with no additive present. When PEG and THF are used, there is a significant effect on the diffusion coefficient with a greater effect observed for THF. These values can be correlated with the average void and interconnect diameters obtained.

The change in the diffusion coefficient of water over time ($\Delta D_w$) can give a possible insight into the effect of each co-solvent on the emulsion (Figure 8). In the presence of THF and PEG, there is greater increase in $D_w$ compared to the emulsion with no co-solvent present. This suggests that both THF and PEG enhance the rate of water diffusion in the emulsion, which would increase Ostwald ripening and is a possible explanation for the increase in void size. The octanol/water partition coefficient value (log $P_{ow}$) for the additives (THF = 0.45; methanol = -0.77) indicates that THF partitions more into the oil phase than methanol, resulting in a less stable emulsion in the presence of THF.

Since there was no significant increase in $D_w$ in the presence of methanol, other effects such as coalescence must be taken into account to explain the observed increase in void diameter. Coalescence can be promoted by dilution of the interfacial layer and subsequent migration of surfactant into the bulk phase due to its increased solubility in the presence of the co-solvent. If this is the case, the surfactant concentration ($C_s$) should have an effect on the final morphology of the material.

To investigate the influence of $C_s$ on morphology, PolyHIPE materials with different $C_s$ values were prepared using THF and methanol as additives. Since THF had been shown to enhance water diffusion, this would allow us to investigate whether the morphology obtained with THF was solely due to Ostwald ripening or whether surfactant depletion from the interface was also involved. Methanol, on the other hand, was shown to have no influence on the rate of water diffusion. Therefore, it was expected that an increase in $C_s$ would have a profound effect on morphology if methanol was influencing the surfactant concentration at the interface. It can be observed from the SEM images (Figure 9a, b) that, as the surfactant concentration in THF containing HIPEs is increased, there is an increase in the open nature of the material but no real discernible effect on void diameter. From the void distribution chart, however, there is a small shift to lower void diameter with increasing surfactant concentration (Figure 10a). However, no flattening or broadening of the distribution is observed with decreasing surfactant concentration.

With THF as additive, the <d>/<D> value (Table 3) increases as the surfactant concentration is increased. This is caused by a slight reduction in <D> with little effect on <d> and provides further evidence that, as the surfactant concentration is increased in the presence of the THF, the open nature of the material increases. However, although there is a slight decrease in the average void size with increasing surfactant concentration, there is a still a significant increase in <D> relative to the material prepared with no additive present (compare entry 3 in Table 3 with entry 1 in Table 1). This suggests that Ostwald ripening is the dominant effect in determining the morphology of polystyrene-based PolyHIPE materials prepared in the presence of THF.

The surfactant concentration was also increased in the presence of methanol in the aqueous phase. This had little effect on the morphology of the resulting materials (Figure 9c, d). From the void size distribution plots (Figure 10b) there is little difference in the distribution when the surfactant concentration is increased from 20 to 30 % w/w. The only discernible effect occurs when the surfactant concentration was increased to 30 % w/w, at which there is a small increase in the percentage of voids present with a diameter of between 30 and 40 $\mu$m and a decrease in the percentage of voids present at higher diameters. However, Table 3 indicates that a maximum value of <D> is obtained when $C_s$ = 25 %.

The surfactant concentration has little effect on the interconnect diameter when THF is used as the additive (Figure 11a). In contrast, increasing the surfactant concentration from 20 to 25 % w/w results in a peak shift towards larger interconnect diameters (Figure 11b) in the presence of methanol. However, when the surfactant concentration is increased from 25 % to 30 % w/w smaller interconnect diameters are obtained, although the <d>/<D> ratios (Table 3) for both materials are similar since this is also accompanied by a decrease in <D>.

From these results, we conclude that Ostwald ripening is not the cause of the increased void diameter in the presence of methanol, since this additive has no influence on the rate of water self-diffusion. Another process by which water can be transported from droplet to droplet is in the interior of w/o micelles[6], which are known to be present in the continuous phase of HIPEs[7]. An increase in surfactant concentration would increase the number of micelles in the continuous phase, which could enhance water transport between droplets. To explain the results obtained with methanol when the surfactant concentration is increased, we conclude that the added surfactant influences two opposing processes: it replaces the surfactant depleted by methanol, which stabilises the emulsion; and it also increases the number of w/o micelles, enhancing water transport and destabilising the emulsion. Support for this comes from the observed maximum values of <D> and <d> at $C_s$ = 25 %, suggesting that two independent processes are operating. The net effect is that there is little observed change in the morphology of PolyHIPEs prepared with methanol when the surfactant content is increased from 20 to 30 % (w/w).

In conclusion, it has been shown that different methods can be used to control emulsion stability to produce POLYHIPE materials with a wide range of void and interconnect sizes. Controlling these parameters will allow the production of different scaffold structures, each tailored and customised toward use in the 3D culture of different cell types.

## EXAMPLE 3

The ability to control the structure of polyHIPE materials is critical to ensure the optimal growth of cultured cells in a 3-dimensional fashion. We have developed a novel application of these materials for this purpose by engineering styrene-based polymeric scaffolds into thin membranes suitable for routine cell growth in vitro by adapting their use to existing tissue culture plastic-ware (see examples: Figures 12 and 13). The approach to using thin layers with large surface areas allows: (1) Good access of the cells into the structure of the material by either static or dynamic seeding; allows good access of oxygen and nutrients (in some cases from both sides of the membrane - see Figure 12, example 1) and removal of waste materials and carbon dioxide therefore minimising the chance of necrosis occurring as found in polymer scaffolds of larger dimensions. These attributes promote the viability of the cultured cells in 3-dimensions; (2) Good access by exogenous reagents (for example, test compounds) to cells growing in 3-dimensions; (3) Cells to be removed from the scaffold after 3-dimensional cell growth for further analysis using enzymatic treatments such as incubation with trypsin (data not shown); (4) Cells and tissues growing within the scaffold may be visualised using electron or optical microscopy (Figures 14 and 20. respectively).

The ability to control the dimensions that constitute the structure of the polymeric material is essential for optimisation of cell growth and behaviour. Subtle differences in the porosity of these materials have significant implications on the ability of cells to adhere to the scaffold, proliferate, differentiate and function (Figures 15-17). Styrene-based polymeric materials produced with 90% pore volume and optimised for in vitro cell growth also enhance cell proliferation, differentiation and function compared to conventional 2-dimensional cell culture plastic-ware (Figures 18-24).

## EXAMPLE 4

*Morphological characteristics of HepG2 cells grown on alternative substrates:*

Scanning electron microscopy revealed significant differences in the appearance of HepG2 cells cultured either on 2-D or 3-D substrates (Figure 25). Cells grown on 2-D planar surfaces formed flat extended structures after 7 days. In general, 2-D cultures appeared heterogeneous and disorganised. After 14 days, cells cultured on tissue culture plastic started to cluster and form aggregates. In some areas of 2-D cultures grown for 14-21 days, HepG2 cells appeared unhealthy, some were rounding up and others were disintegrating (data not shown). Cells cultured on 3-D polystyrene, spread across and into the structure of the scaffold. Cells initially clustered into colonies of closely packed cells within the substance of the polymer, resembling small multi-cellular aggregates. This was indicative of the greater attachment and interaction between adjacent cells growing on the scaffold. After 7 days, cultures of HepG2 cells grown in 3-D appeared more homogeneous than their 2-D counterparts. Growing cultures at lower seeding densities showed that cells attached to the scaffold and extended across voids. This demonstrated how cells grown in 3-D can maximise their surface area by interacting with adjacent cells and the incubation medium. In addition, higher magnification imaging of individual cells grown in 3-D revealed a significantly greater number of micro-villi compared to cells grown on 2-D surfaces.

Transmission electron microscopy was used to examine the ultra-structural features of HepG2 cells cultured on different materials (Figure 26). In general, analysis of intact whole cells grown on either 2-D or 3-D substrates contained a range of organelles typical of most mammalian cells, including mitochondria, nuclei, endoplasmic reticulum and lipid droplets. Ultra-thin sections of cell preparations grown in 3-D showed clearly how cells grow around and in close association with the polystyrene scaffold. Cells cultured on the 3-D surfaces displayed numerous morphological features typical of the liver tissue. Nuclear membranes appeared to be normal. Numerous mitochondria visualised displayed structural variances within the normal range. No specific pathological alterations were detectable in either the smooth or the rough endoplasmic reticulum, in the Golgi complexes, or in the glycogen content. The presence of these ultra-structural features indicated that HepG2 cells grown on 3-D substrates were metabolically active. The presence of peroxisomal clusters (Figure 26B), which are ubiquitous cell organelles abundant in mammalian liver and kidney, was particularly encouraging. Liver peroxisomes are known to be responsible for the β-oxidation of the side chain of cholesterol in the course of bile acid synthesis, a pathway associated with differentiated hepatocytes [8].

In native liver tissue, hepatocytes possess polarity with two or three basal surfaces facing the sinusoid while adjacent cells form the bile canaliculi. Micrographs of cells grown in 3-D revealed adjacent hepatocytes often shared microvilli-lined channels lined with tight junctions. This observation suggests that cultured HepG2 cells may be polarized and capable of forming channels that resemble bile canaliculi [9]. These structures are known to be rich in microvilli and components of bile metabolised in the cells are normally secreted into the canaliculi.

## EXAMPLE 5

*Enhanced cell viability during 3-D cell growth:*

We examined whether the surfaces used in this study were biocompatible to support the growth of viable HepG2 cells. Figure 27A illustrates the MTT absorbance values for hepatocytes grown on 2-D control surfaces and our 3-D scaffolds. Viable cells were successfully cultured on both substrates for up to 21 days. The assay revealed that cell viability was significantly enhanced when grown in 3-D. It should be noted that cells grown on scaffolds have a greater surface area on which to attach and grow, compared to planar surfaces, where space per cell is restricted. Where possible, this difference has been taken into account and values were normalised for *in vitro* assays.

**EXAMPLE 6**

*Enhanced cell metabolism during 3-D cell growth:*

The metabolic activity of HepG2 cells was assessed by determination of the level of albumin secretion. Figure 27B shows the time courses of albumin secretion on 2-D tissue culture plastic and the 3-D scaffolds. Values have been normalised to account for any differences in cell number. It can be seen clearly that there is a significantly higher albumin concentration in cultures grown on 3-D surfaces compared to cells grown in 2-D for all the time points that were tested. Albumin levels in cultures grown on flat tissue culture plastic peaked at day 14 and then decreased rapidly at 21 days. This did not occur in cultures grown on 3-D scaffolds indicating that the 3-D environment is more conducive to cell function.

**EXAMPLE 7**

*Effects of methotrexate on cells grown in 2-D and 3-D:*

HepG2 cells grown in 2-D and 3-D formats were treated with various concentrations of MTX to evaluate their tolerance to a well known cytotoxin. Following each treatment period, cultures were then studied for biochemical (Figure 28) and morphological changes (Figure 29 & 30).

Figure 28A illustrates cell viability after 1 and 7 days treated with varying concentrations of MTX. Treatment of HepG2 monolayers with MTX resulted in a gradual increase of absorbance at $15\mu$M MTX after 24 hours but absorbance levels started to drop at 7 days (data not shown). With increasing MTX concentrations, the viability of cells grown on 2-D surfaces was visibly reduced especially at the higher levels of the cytotoxin. In 3-D cultures, sensitivity to MTX was not evident in the lesser concentrations of MTX; only at $62\mu$M MTX was there a significant decrease in absorbance levels compared to control values. This pattern was also seen in 3-D cultures grown for 7 days, whereas cells grown in monolayers for 7 days showed a sharp decrease in absorbance levels at $125\mu$M MTX concentrations. These results imply that cells cultured on 2-D surfaces under these conditions remain viable for a shorter period compared to cells cultured on 3-D scaffolds.

The metabolic activity of HepG2 cells was significantly reduced by increasing concentrations of MTX (Figure 28B). Cells grown in 2-D were sensitive to the lowest concentrations of MTX tested ($8\mu$M), whereas this level did not significantly influence albumin secretion by cells grown in 3-D (data not shown). However, increased levels of MTX ($15.6\mu$M) did begin to reduce albumin secretion by cells cultured on scaffolds. Throughout cytotoxic challenge, higher levels of albumin secretion were noted in cells grown on 3-D plastic compared to 2-D materials. These data illustrate that hepatocyte cell function was impaired in the presence of MTX in a dose-dependent manner and cells grown in 3-D appeared more tolerant to the cytotoxin.

Measurement of transglutaminase was performed as a test for HepG2 cell toxicity in response to increasing concentrations of MTX. We examined the effects of MTX on transglutaminase levels in 2-D and 3-D cultures after 1, 7 and 10 days exposure to the cytotoxin. (Figure 28C). In control cultures, where there was no addition of MTX, levels of transglutaminase were found to be minimal and at similar levels. With increasing concentrations of the drug, such as $31\mu$M MTX, 2-D cultures secreted significantly higher levels of transglutaminase which increased in a dose dependent manner unlike cells grown in 3-D culture. These differences were statistically significant in all 2-D cultures at 7 and 10 days compared to their 3-D counterparts. In the 3-D cultures, increasing the concentration of MTX did not cause a significant increase in transglutaminase levels although there was a gradual rise in enzyme levels secreted into the culture medium at higher concentrations of the drug. These data further suggest that cells on 3-D porous materials are more tolerant to increasing levels of cytotoxin challenge.

Scanning (SEM) and transmission electron micrographs (TEM) demonstrated concentration dependent changes in cell structure subsequent to treatment with MTX. Representative examples of such morphological changes are illustrated in Figures 29 and 30. Normal, healthy HepG2 cells express numerous micro-villi on their cell surface. When challenged with MTX, cells grown on 2-D surfaces gradually lost their micro-villi in a dose dependent manner, whilst cells grown on scaffolds continued to express this structural feature (Figure 29). In response to increasing levels of MTX, the surface of HepG2 cells grown as monolayers, first decreased the numbers of micro-villi, then became flattened, and then started to disintegrate. No such changes were observed to the structure of HepG2 cells grown on 3-D scaffolds, although the micro-villi of cells grown in the highest concentration of MTX tested ($125\mu$M), did begin to show signs of flattening.

Further examination by TEM revealed ultra-structural changes to cells challenged by the cytotoxin (Figure 30).

Cells grown on 2-D surfaces possessed healthy morphology with prominent nuclei with visible nucleoli in control cultures. Following treatment with 8μM MTX, cells with good nuclear architecture remained visible in 2-D cultures although areas of cellular necrosis were also evident. Hepatocyte 2-D cultures treated with increasing levels of MTX showed marked cytotoxicity and most cells became necrotic at high levels of MTX. Features such as endoplasmic reticulum de-granulation and the presence of ribosomal ghosts were observed. Furthermore, the granular cytoplasm generally lacked an organised structure and clumped chromatic granules were dispersed throughout the nucleus. Sub-cellular evidence of apoptosis was also observed; the plasma membrane was seen to rupture and a marked amount of vacuolation, possibly reflecting presence of lipid droplets and cellular degeneration. Cell shrinkage was also obvious, as well as a loss of cell-to-cell contact followed by formation of apoptotic bodies (autophagolysosomes) and cell death. At the highest concentration, 'ghosts' of cellular remains were observed, indicating that cells grown in 2-D exposed to higher levels of MTX had undergone an advanced stage of cell death.

In contrast, HepG2 cells grown in 3-D culture and exposed to MTX were significantly more resistant to the effects of the cytotoxin. The ultra-structure of cells treated with lower concentrations of MTX possessed normal organelles in their cytoplasm (RER, ribosomes, mitochondria and lipid droplets). The nuclei displayed normal heterochromatin and nucleoli. These features were well preserved throughout most of the concentrations of MTX tested. However, in some cells in the presence of 125μM of MTX, the nuclear membrane had an irregular morphology and other sub-cellular features, such as mitochondria, which appeared to be slightly abnormal. It is likely therefore at higher concentrations of MTX, cells in 3-D cultures are starting to undergo changes similar to those experienced by cells cultured in 2-D as seen significantly lower concentrations of the cytotoxin.

In conclusion, the growth of cells on styrene-based polymeric scaffolds adapted for use in existing cell culture plastic-ware provides the opportunity for 3-dimensional cell growth in vitro. Cell behaviour is influenced by the environment in which cells grow and cell growth in 3-dimensions is more realistic and more closely resembles the growth conditions cells normally experience in the body. The apparatus described herein provides an opportunity for researchers to routinely grow cells in 3-dimensions which will be invaluable for more accurate read-outs from cell models and assays. The apparatus is also inert, easy to use, can be sterilised, is cheap to manufacture and produce, it is robust and reproducible, has an indefinite shelf-life and is adaptable to many applications.

## EXAMPLE 8

In a further application for the use of the polystyrene scaffold, we have developed an organotypic model of mammalian skin consisting of a stratified sheet of epidermal keratinocytes grown at the media/air interface on a layer of dermal fibroblasts in the presence or absence of a collagen gel or solution-coating within the scaffold. This system enables long term growth and maintenance of polarised epithelia that closely resemble native skin. The technology can be used to investigate the function of skin epithelial cells in a broad range of applications, including basic science, development of pharmaceuticals and assessment of compound toxicity.

Organotypic models for the growth of mammalian skin are well established and a number of procedures have been developed to achieve this in vitro (for example: Bohnert et al. 1986; Ikuta et al. 2006; Prunieras et al. 1983; Schoop et al. 1999). The existing procedures requires the growth of dermal fibroblasts within a collagen gel mixture, upon which keratinocytes are seeded in a two layered sandwich. The gel shrinks over time; it is then raised to the air/media interface to enable changes in cell growth and activity. Handling the gel is tricky and requires time, skill and concentration. As a consequence this model is not readily adaptable for high throughput screening strategies or in circumstances where a reduction in variability is required and ease of handling is needed.

Here we demonstrate the application of our 3D polystyrene scaffolds to more readily enable the routine use and handling of dermal fibroblasts and collagen gels for organotypic skin cocultures. In brief, cultures of dermal fibroblasts are seeded onto the surface of our 3D polystyrene scaffolds in appropriate growth media (Figure 31a). The cells grow over the surface and into the structure of the 3D membrane (Figure 32). This can be achieved in the presence or absence of a collagen solution/gel or pre-coating of the scaffold with collagen solution (e.g. Type I collagen). The inert 3D plastic scaffold provides support for the cultured fibroblasts. The scaffolds laiden with fibroblasts are readily handled and can be transferred into fresh cell culture plastic ware (e.g. 6-welled plate) if required. In addition, shrinkage of a cast collagen gel is minimised which reduces variability between experiments. Subsequent to the establishing the fibroblast culture, an organotypic coculture is set up by seeding epidermal keratinocytes (e.g. HaCaT cells) onto the surface of the fibroblasts (Figure 31b). When the keratinocyte culture is established (~2 days), the surface of the polystyrene scaffold is raised to the air-liquid interface. Air exposure induces stratification of the keratinocytes (Figures 31c and 33).

The advantages for using the 3D porous polystyrene scaffolds for organotypic coculture of mammalian skin are:

- To provide support for the cells (and gel if appropriate) and 3D environment for the dermal fibroblast culture
- To enable ease of handling of the fibroblast culture / collagen gel mix and avoid breakage or damage to the gels/culture
- To minimise shrinkage of the collagen gel

- To enable freedom to readily transfer organotypic cultures to other vessels
- To raise the culture to the air/liquid interface either by reducing the media level or by raising the scaffold itself (for example, using the well insert configuration together with adaptors to increase the height of the insert)

**References**

1. A. Barbetta and N. R. Cameron, Macromolecules, 2004, 37, 3188.

2. A. S. Kabalnov and E. G. Shchukin, Adv. Colloid Interface. Sci., 1992, 38, 69.

3. K. J. Lissant (ed.), 'Emulsions and Emulsion Technology Part 1', Marcel Dekker Inc., New York, 1974.

4. M. P. Aronson and M. F. Petko, J. Colloid Interface Sci., 1993, 159, 134.

5. M. W. Hayman, K. H. Smith, N. R. Cameron, and S. A. Przyborski, Biochem. Biophys. Res. Commun., 2004, 314, 483; M. W. Hayman, K. H. Smith, N. R. Cameron, and S. A. Przyborski, J. Biochem. Biophys. Methods, 2005, 62, 231.

6. J. G. Weers, in 'Modern Aspects of Emulsion Science', ed. B. P. Binks, RSC, Cambridge, 1998.

7. J. C. Ravey and M. J. Stebe, Prog. Colloid Polym. Sci., 1990, 82, 218; R. Pons, P. Erra, C. Solans, J. C. Ravey, and M. J. Stebe, J. Phys. Chem., 1993, 97, 12320; R. Pons, J. C. Ravey, S. Sauvage, M. J. Stebe, P. Erra, and C. Solans, Colloids Surf., A: Physicochem. Eng. Aspects, 1993, 76, 171; J. C. Ravey, M. J. Stebe, and S. Sauvage, Colloids Surf., A: Physicochem. Eng. Aspects, 1994, 91, 237.

8. Beier K, Fahimi HD. Application of automatic image analysis for quantitative morphological studies of peroxisomes in rat liver in conjunction with cytochemical staining with 3-3'-diaminobenzidine and immunocytochemistry. Microsc Res Tech 1992 Jun 1;21(4):271-282.

9. Abu-Absi SF, Friend JR, Hansen LK, Hu WS. Structural polarity and functional bile canaliculi in rat hepatocyte spheroids. Exp Cell Res 2002 Mar 10;274(1):56-67.

10. Bohnert A, Hornung J, Mackenzie IC, Fusenig NE (1986). Epithelialmesenchymal interactions control basement membrane production and differentiation in cultured and transplanted mouse keratinocytes. Cell Tissue Res 244:413-429.

11. Ikuta S, Sekino N, Hara T, Saito Y, Chida K (2006). Mouse epidermal keratinocytes in three-dimensional organotypic coculture with termal fibroblasts form a stratified sheet resembling skin. Biosci Biotechnol Biochem 70:2669-2675.

12. Prunieras M, Regnier M, Woodley D (1983). Methods for cultivation of keratinocytes with an air-liquid interface. J Invest Dermatol 81:28s-33s.

13. Schoop VM, Mirancea N, Fusenig NE (1999). Epidermal organisation and differentiation of HaCaT keratinocytes in organotypic coculture with human dermal fibroblasts. J Invest Dermatol 112:343-353.

Table 1

| Temp$_{aq}$[a]/°C | Additive (%)[b] | <D>/μm[c] | <d>/μm[d] | <d>/<D> |
|---|---|---|---|---|
| 23 | - | 35 | 11 | 0.29 |
| 50 | - | 74 | 16 | 0.27 |
| 60 | - | 94 | 19 | 0.22 |
| 80 | - | 104 | 26 | 0.25 |
| 23 | PEG (0.2) | 82 | 12 | 0.15 |

(continued)

| Temp$_{aq}$[a]/°C | Additive (%)[b] | <D>/μm[c] | <d>/μm[d] | <d>/<D> |
|---|---|---|---|---|
| 23 | PEG (0.4) | 73 | 14 | 0.19 |
| 23 | PEG (0.8) | 84 | 15 | 0.17 |
| 23 | PEG (1.5) | 74 | 16 | 0.22 |
| 23 | MeOH (1.0) | 59 | 12 | 0.20 |
| 23 | MeOH (2.0) | 57 | 12 | 0.21 |
| 23 | MeOH (3.0) | 68 | 16 | 0.24 |
| 23 | MeOH (4.0) | 65 | 14 | 0.22 |
| 23 | THF (0.4) | 60 | 12 | 0.20 |
| 23 | THF (0.8) | 90 | 12 | 0.13 |
| 23 | THF (1.0) | 72 | 12 | 0.17 |
| 23 | THF (1.5) | 99 | 15 | 0.15 |

[a] aqueous phase temperature
[b] aqueous phase additive expressed as vol. % (wt./vol. % for PEG)
[c] average void diameter determined by SEM
[d] average interconnect diameter determined by Hg porosimetry

Table 2

| Additive (%)[b] | <D>/μm [c] | <d>/μm [d] | $(D_{wi}/m^2s^{-1}) \times 10^{-10}$[e] | $(D_{wf}/m^2s^{-1}) \times 10^{-10}$[f] | $(\Delta D_w/m^2s^{-1}) \times 10^{-10}$[g] |
|---|---|---|---|---|---|
| - | 35 | 11 | 7.1 | 8.2 | 1.1 |
| THF (1.5) | 99 | 15 | 10.1 | 12.1 | 2 |
| MeOH (2) | 57 | 12 | 7.3 | 8.1 | 0.8 |
| PEG (1.5) | 74 | 16 | 7.8 | 9.4 | 1.6 |

[a]In each case the aqueous phase was kept at room temperature during emulsion preparation.
[b] aqueous phase additive expressed as % (v/v) (% (w/v) for PEG)
[c] average void diameter determined by SEM
[d] average void diameter determined by Hg porosimetry
[e] $D_{wi}$ = initial value of water self-diffusion coefficient
[f] $D_{wf}$ = final value of water self-diffusion coefficient
[g] $\Box D_w$ = change in water self-diffusion coefficient

Table 3

| Additive[a] | $C_S$ (% w/w)[b] | <D>/μm[c] | <d>/μm[d] | <d>/<D> |
|---|---|---|---|---|
| THF | 20 | 74 | 12 | 0.16 |
| THF | 25 | 72 | 12 | 0.17 |
| THF | 30 | 66 | 14 | 0.22 |
| MeOH | 20 | 58 | 10 | 0.17 |
| MeOH | 25 | 65 | 15 | 0.23 |

(continued)

| Additive[a] | $C_S$ (% w/w)[b] | $<D>/\mu m$[c] | $<d>/\mu m$[d] | $<d>/<D>$ |
|---|---|---|---|---|
| MeOH | 30 | 53 | 12 | 0.23 |

[a] 1.5 % (v/v) THF, 4 % (v/v) methanol
[b] Concentration of surfactant (Span 80) expressed as percentage of total monomer phase
[c] average void diameter determined by SEM
[d] average interconnect diameter determined by Hg porosimetry

**Claims**

1. A cell culture substrate comprising a plurality of a sectioned polymerised high Internal phase emulsion polymer wherein said sections are 50-1000 microns and wherein the pore volume of the polymerised high internal phase emulsion polymer is between 88% and 92%.

2. A substrate according to claim 1 wherein said substrate comprises a hydrophobic elastomer at a concentration of between 20 %(w/w) and 40% (w/w)..

3. A substrate according to claim 2 wherein said elastomer is selected from the group consisting of: 2-ethylhexyl acrylate; n-butyl acrylate and n-hexyl acrylate.

4. A substrate according to any of claims 1-3 wherein said cell culture substrate comprises a surfactant.

5. A substrate according to claim 4 wherein said surfactant is provided at a concentration of 20-30% (w/w), or between 24-26 %(w/w, or around 25%(w/w).

6. A process for the formation of a high internal phase polymer having a pore volume of between 88% and 92% comprising the steps of:

   i) forming a preparation comprising an high internal phase emulsion comprising a hydrophobic elastomer at a concentration of between 20% (w/w) and 40% (w/w) and a surfactant that is provided at a concentration of 20-30% (w/w);
   ii) forming a preparation comprising a catalyst;
   iii) combining the preparations in (i) and (ii);
   iv) incubating the combined preparation to allow formation of a high internal phase emulsion polymer; and
   v) sectioning said polymer into parts that are 50-1000 microns thick.

7. A method for the culture of cells comprising the steps of:

   i) providing a cell culture vessel comprising:

      a) cells;
      b) a cell culture substrate according to any of claims 1- 5;
      c) cell culture medium sufficient to support the growth of said cells; and

   ii) providing cell culture conditions which promote the proliferation and/or differentiation of said cells.

8. A cell culture vessel comprising a cell culture substrate according to any of claims 1-5.

9. A method to screen for an agent wherein said agent affects the proliferation, differentiation or function of a cell comprising the steps of:

   i) providing cell culture comprising at least one cell and a cell culture substrate according to any of claims 1-5;
   ii) adding at least one agent to be tested; and
   iii) monitoring the activity of the agent with respect to the proliferation, differentiation or function of said cells.

10. A method for the identification of genes associated with cell differentiation comprising the steps of:

   i) providing cell culture comprising at least one cell and a cell culture substrate according to any of claims 1-5
   ii) extracting nucleic acid from cells contained in said cell culture;
   iii) contacting said extracted nucleic acid with a nucleic acid array; and
   iv) detecting a signal which indicates the binding of said nucleic acid to a binding partner on said nucleic acid array.

11. An *in vitro* method to analyse the development of cancerous cells from normal cells comprising

   i) forming a preparation comprising a cell culture substrate according to any of claims 1- 5 including cells;
   ii) adding at least one agent capable of inducing cell transformation; and
   iii) monitoring the effect, or not, of said agent on the transformation of said cells.

12. The use of a substrate comprising high internal phase emulsion polymer according to any of claims 1-5 to culture cells.

13. The use of a substrate comprising a high internal phase emulsion polymer according to any of claims 1-5 to determine the liver toxicity of an agent.

14. A method to test the liver toxicity of an agent comprising the steps of:

   i) providing a cell culture comprising at least one hepatocyte cell and a cell culture substrate according to any of claims 1- 5;
   ii) adding at least one agent to be tested; and
   iii) monitoring the activity of the agent with respect to the proliferation, differentiation or function of said hepatocyte cells as a measure of toxicity of the agent.

15. A method for the growth and differentiation of a keratinocyte and/a keratinocyte precursor stem cell comprising:

   i) forming a preparation comprising a cell culture substrate according to any of claims 1-5, fibroblast feeder cells and cell culture medium;
   ii) culturing said feeder cells to provide a cell culture substrate that is substantially coated with said feeder cells;
   iii) contacting said coated substrate with keratinocytes and/or keratinocyte precursor stem cells; and
   iv) culturing the combined cell preparation under conditions conducive to the growth and differentiation of said keratinocytes and/or keratinocyte precursor stem cells.

16. A method to test an agent comprising:

   i) forming a preparation comprising a cell culture substrate according to any of claims 1-5 which includes an agent to be tested;
   ii) monitoring the effect of said agent on keratinocyte cell growth and/or differentiation when compared to a control preparation that does not indude said agent.

17. An apparatus for the culture of cells comprising a cell culture substrate according to any of claims 1-5. a cell culture vessel and an insert adapted to co-operate with said cell culture vessel and contain said cell culture substrate and said cells.

18. The use of a cell culture substrate according to any of claims 1-5 for the preparation of differentiated skin composite.

**Patentansprüche**

1. Ein Zellkultursubstrat, das eine Pluralität eines zerlegten polymerisierten High Internal Phase-Emulsionspolymerisats enthält, wobei besagte Teile 50-1000 Mikrometer groß sind, und worin das Porenvolumen des polymerisierten High Internal Phase-Emulsionspolymerisats zwischen 88% und 92% beträgt.

2. Ein Substrat gemäß Anspruch 1, wobei besagtes Substrat ein hydrophobes Elastomer mit einer Konzentration zwischen 20% (w/w) und 40% (w/w) enthält.

**3.** Ein Substrat gemäß Anspruch 2, wobei besagtes Elastomer aus der Gruppe, bestehend aus 2-Ethylhexylacrylat, n-Butylacrylat und n-Hexylacrylat, gewählt wird.

**4.** Ein Substrat gemäß jedwedem der Ansprüche 1-3, wobei besagtes Zellkultursubstrat eine oberflächenaktive Substanz enthält.

**5.** Ein Substrat gemäß Anspruch 4, wobei besagte oberflächenaktive Substanz in einer Konzentration von 20-30% (w/w) oder zwischen 24-26% (w/w) oder ungefähr 25% (w/w) bereitgestellt wird.

**6.** Ein Verfahren zur Bildung eines High Internal Phase-Polymers, welches ein Porenvolumen zwischen 88% und 92% aufweist, und die folgenden Schritte umfasst:

i) Bilden eines Präparats, das eine High Internal Phase-Emulsion enthält, welches ein hydrophobes Elastomer in einer Konzentration von zwischen 20% (w/w) und 40% (w/w) sowie eine oberflächenaktive Substanz enthält, die in einer Konzentration von 20-30% (w/w) bereitgestellt wird;
ii) Bilden eines Präparats, das einen Katalysator enthält;
iii) Vereinen der Präparate von (i) und (ii);
iv) Inkubieren des vereinigten Präparats, um die Bildung eines High Internal Phase-Emulsionspolymerisats zu ermöglichen; und
v) Zerlegen besagten Polymers in Teile, die 50-1000 Mikrometer dick sind.

**7.** Ein Verfahren für die Züchtung von Zellen, bestehend aus folgenden Schritten:

i) Bereitstellen eines Zellkulturgefäßes, welches Folgendes enthält:

a) Zellen,
b) Ein Zellkultursubstrat gemäß jedwedem der Ansprüche 1-5;
c) Zellkulturmedium, welches ausreicht, um das Wachstum besagter Zellen zu unterstützen; und

ii) Bereitstellen von Zellkulturbedingungen, die die Proliferation und/oder Differenzierung besagter Zellen fördern.

**8.** Ein Zellkulturgefäß, welches ein Zellkultursubstrat gemäß jedwedem der Ansprüche 1-5 enthält.

**9.** Ein Verfahren, um auf einen Wirkstoff hin zu untersuchen, worin besagter Wirkstoff die Proliferation, Differenzierung oder Arbeitsweise einer Zelle beeinflusst. Dieses umfasst folgende Schritte:

i) Bereitstellen einer Zellkultur, die mindestens eine Zelle und ein Zellkultursubstrat gemäß jedwedem der Ansprüche 1-5 umfasst;
ii) Hinzufügen mindestens eines zu prüfenden Wirkstoffs; und
iii) Überwachen der Aktivität des Wirkstoffs im Hinblick auf die Proliferation, Differenzierung oder Arbeitsweise besagter Zellen.

**10.** Ein Verfahren für die Bestimmung von Genen, die mit der Zelldifferenzierung in Zusammenhang stehen, bestehend aus folgenden Schritten:

i) Bereitstellen einer Zellkultur, die mindestens eine Zelle und ein Zellkultursubstrat gemäß jedwedem der Ansprüche 1-5 enthält;
ii) Extrahieren von Nucleinsäure aus Zellen, die in besagter Zellkultur enthalten sind;
iii) Besagte extrahierte Nucleinsäure mit einem Nucleinsäure-Array in Verbindung bringen; und
iv) Detektieren eines Signals, welches das Binden besagter Nucleinsäure an einen Bindungspartner an besagtem Nucleinsäure-Array anzeigt.

**11.** Ein *in vitro*-Verfahren zur Analyse der Entwicklung karzinöser Zellen aus normalen Zellen, das Folgendes umfasst:

i) Bilden eines Präparats, das ein Zellkultursubstrat gemäß jedwedem der Ansprüche 1-5 einschließlich Zellen enthält;
ii) Hinzufügen mindestens eines Wirkstoffs, der in der Lage ist, eine Zelltransformation herbeizuführen; und

iii) Überwachen der Auswirkung bzw. Nichtauswirkung besagten Wirkstoffs auf die Transformation besagter Zellen.

**12.** Der Einsatz eines Substrats, das ein High Internal Phase-Emulsionspolymerisat gemäß jedwedem der Ansprüche 1-5 enthält, an Kulturzellen.

**13.** Der Einsatz eines Substrats, das ein High Internal Phase-Emulsionspolymerisat gemäß jedwedem der Ansprüche 1-5 enthält, um die Lebertoxizität eines Wirkstoffs zu bestimmen.

**14.** Ein Verfahren zur Prüfung der Lebertoxizität eines Wirkstoffs, welches folgende Schritte umfasst:

i) Bereitstellen einer Zellkultur, die mindestens eine Hepatozytzelle und ein Zellkultursubstrat gemäß jedwedem der Ansprüche 1-5 enthält;
ii) Hinzufügen mindestens eines zu prüfenden Wirkstoffs; und
iii) Überwachen der Aktivität des Wirkstoffs im Hinblick auf die Proliferation, Differenzierung oder Arbeitsweise besagter Hepatozytzellen als ein Maßstab für die Toxizität des Wirkstoffs.

**15.** Ein Verfahren für Wachstum und Differenzierung eines Keratinozyts und/oder einer Keratinozyten-Vorläuferstammzelle, welches folgendes umfasst:

i) Bilden eines Präparats, das ein Zellkultursubstrat gemäß jedwedem des Ansprüche 1-5, Fibroblast-Fütterzellen und Zellkulturmedium enthält;
ii) Züchten besagter Fütterzellen, um ein Zellkultursubstrat zu erbringen, welches mit besagten Fütterzellen reichlich belegt ist;
iii) Besagtes belegtes Substrat mit Keratinozyten und/oder einer Keratinozyten-Vorläuferstammzellen in Verbindung bringen; und
iv) Züchten des vereinten Zellpräparats unter Bedingungen, die für Wachstum und Differenzierung besagter Keratinozyten und/oder einer Keratinozyten-Vorläuferstammzellen förderlich sind.

**16.** Ein Verfahren zur Prüfung eines Wirkstoffs, welches Folgendes umfasst:

i) Bilden eines Präparats, das ein Zellkultursubstrat gemäß jedwedem der Ansprüche 1-5 enthält, welches einen zu prüfenden Wirkstoff beinhaltet;
ii) Überwachen der Wirkung besagten Wirkstoffs auf das Keratinozytenwachstum und/oder Differenzierung, wenn mit einem Kontrollpräparat verglichen wird, welches besagten Wirkstoff nicht enthält.

**17.** Ein Gerät für das Züchten von Zellen, welches Folgendes enthält: ein Zellkultursubstrat gemäß jedwedem der Ansprüche 1-5, ein Zellkulturgefäß und einen Einsatz, der angepasst ist, um mit besagtem Zellkulturgefäß zusammenzuwirken und besagtes Zellkultursubstrat und besagte Zellen zu enthalten.

**18.** Die Verwendung eines Zellkultursubstrats gemäß jedwedem der Ansprüche 1-5 zur Gewinnung von differenzierter Hautverbundstruktur.

**Revendications**

**1.** Un substrat de culture cellulaire comprenant une pluralité de polymères en émulsion à phase interne élevée polymérisés sectionnés où lesdites sections sont de 50 à 1000 microns et où le volume de pore du polymère en émulsion à phase interne élevée polymérisé se situe entre 88% et 92%.

**2.** Un substrat selon la Revendication 1 où ledit substrat contient un élastomère hydrophobe à une concentration située entre 20% (poids/poids) et 40% (poids/poids).

**3.** Un substrat selon la Revendication 2 où ledit élastomère est sélectionné dans le groupe se composant de : acrylate de 2-éthylehexyle, acrylate de n-butyle et acrylate de n-hexyle.

**4.** Un substrat selon l'une quelconque des Revendications 1 à 3 où ledit substrat de culture cellulaire contient un agent tensioactif.

**5.** Un substrat selon la Revendication 4 où ledit agent tensioactif est fourni à une concentration de 20 à 30% (poids/poids) ou entre 24 et 26% (poids/poids) ou autour de 25% (poids/poids).

**6.** Un processus de formation d'un polymère à phase interne élevée possédant un volume de pore situé entre 88% et 92%, comprenant les opérations suivantes :

i) la formation d'une préparation contenant une émulsion à phase interne élevée contenant un élastomère hydrophobe à une concentration située entre 20% (poids/poids) et 40% (poids/poids) et un agent tensioactif qui est fourni à une concentration de 20 à 30% (poids/poids),
ii) la formation d'une préparation contenant un catalyseur,
iii) la combinaison des préparations de (i) et (ii),
iv) l'incubation de la préparation combinée de façon à permettre la formation d'un polymère en émulsion à phase interne élevée, et
v) le sectionnement dudit polymère en parties qui présentent une épaisseur de 50 à 1000 microns.

**7.** Un procédé de culture de cellules comprenant les opérations suivantes :

i) la fourniture d'un récipient pour culture cellulaire contenant :

a) des cellules,
b) un substrat de culture cellulaire selon l'une quelconque des Revendications 1 à 5,
c) un milieu de culture cellulaire suffisant pour prendre en charge la croissance desdites cellules, et

ii) la fourniture de conditions de culture cellulaire qui favorisent la prolifération et/ou la différenciation desdites cellules.

**8.** Un récipient pour culture cellulaire contenant un substrat de culture cellulaire selon l'une quelconque des Revendications 1 à 5.

**9.** Un procédé de criblage destiné à la recherche d'un agent où ledit agent affecte la prolifération, la différenciation ou la fonction d'une cellule comprenant les opérations suivantes :

i) la fourniture d'une culture cellulaire comprenant au moins une cellule et un substrat de culture cellulaire selon l'une quelconque des Revendications 1 à 5,
ii) l'ajout d'au moins un agent à tester, et
iii) la surveillance de l'activité de l'agent par rapport à la prolifération, la différenciation ou la fonction desdites cellules.

**10.** Un procédé d'identification de gènes associés à une différenciation de cellules comprenant les opérations suivantes :

i) la fourniture d'une culture cellulaire contenant au moins une cellule et un substrat de culture cellulaire selon l'une quelconque des Revendications 1 à 5,
ii) l'extraction d'un acide nucléique des cellules contenues dans ladite culture cellulaire,
iii) la mise en contact dudit acide nucléique extrait avec une matrice d'acides nucléiques, et
iv) la détection d'un signal qui indique la liaison dudit acide nucléique à un partenaire de liaison sur ladite matrice d'acides nucléiques.

**11.** Un procédé *in vitro* d'analyse du développement de cellules cancéreuses à partir de cellules normales comprenant

i) la formation d'une préparation contenant un substrat de culture cellulaire selon l'une quelconque des Revendications 1 à 5 contenant des cellules,
ii) l'ajout d'au moins un agent capable d'induire une transformation de cellules, et
iii) la surveillance de l'effet ou non dudit agent sur la transformation desdites cellules.

**12.** L'utilisation d'un substrat contenant un polymère en émulsion à phase interne élevée selon l'une quelconque des Revendications 1 à 5 de façon à cultiver des cellules.

**13.** L'utilisation d'un substrat contenant un polymère en émulsion à phase interne élevée selon l'une quelconque des Revendications 1 à 5 de façon à déterminer la toxicité pour le foie d'un agent.

14. Un procédé de test de la toxicité pour le foie d'un agent comprenant les opérations suivantes :

    i) la fourniture d'une culture cellulaire contenant au moins une cellule hépatocyte et un substrat de culture cellulaire selon l'une quelconque des Revendications 1 à 5,
    ii) l'ajout d'au moins un agent à tester, et
    iii) la surveillance de l'activité de l'agent par rapport à la prolifération, la différenciation ou la fonction desdites cellules hépatocytes en tant que mesure de toxicité de l'agent.

15. Un procédé de croissance et de différenciation d'un kératinocyte et/ou d'une cellule souche de précurseur de kératinocyte comprenant :

    i) la formation d'une préparation contenant un substrat de culture cellulaire selon l'une quelconque des Revendications 1 à 5, des cellules nourricières de fibroblastes et un milieu de culture cellulaire,
    ii) la culture desdits cellules nourricières de façon à fournir un substrat de culture cellulaire qui est sensiblement enduit avec lesdits cellules nourricières,
    iii) la mise en contact dudit substrat enduit avec des kératinocytes et/ou des cellules souches de précurseur de kératinocyte, et
    iv) la culture de la préparation cellulaire combinée dans des conditions propices à la croissance et à la différenciation desdites kératinocytes et/ou cellules souches de précurseur de kératinocyte.

16. Un procédé de test d'un agent comprenant :

    i) la formation d'une préparation contenant un substrat de culture cellulaire selon l'une quelconque des Revendications 1 à 5 qui comprend un agent à tester,
    ii) la surveillance de l'effet dudit agent sur la croissance et/ou la différenciation de cellules de kératinocyte en comparaison d'une préparation témoin qui ne contient pas ledit agent.

17. Un appareil pour la culture de cellules composant un substrat de culture cellulaire selon l'une quelconque des Revendications 1 à 5, un récipient pour culture cellulaire et un insert adapté de façon à coopérer avec ledit récipient pour culture cellulaire et à contenir ledit substrat de culture cellulaire et lesdites cellules.

18. L'utilisation d'un substrat de culture cellulaire selon l'une quelconque des Revendications 1 à 5 pour la préparation d'un composite de peau différencié.

Figure 1

**Figure 2**

Figure 3

Figure 4

Figure 5

**Figure 6**

EP 2 018 418 B1

Figure 7

35

Figure 8

**Figure 9**

Figure 10

Figure 11

Figure 12

EXAMPLE 1

Existing culture ware
(e.g. 6 well multi-plate)

Cell type 1 growing
as a 3D culture within
the scaffold material

3D thin layer scaffold
within existing welled dish

Cell type 1

EXAMPLE 2

Existing culture ware
(e.g. 6 well multi-plate)

Cell culture insert adapted to
hold 3D thin layer scaffold

Cell type 1

Cell type 2

Co-culture

Figure 13

Figure 14

Figure 15

PV 90%       PV 95%

Figure 16

Figure 17

Figure 18

Figure 19

Figure 20

Figure 21

Figure 22

Figure 23

Figure 24

Figure 25

Figure 26

Figure 27

figure 28

Figure 29

Figure 30

Figure 31

Figure 32

Figure 33

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2003014334 A
- WO 0034454 A
- WO 2004005355 A
- WO 2004004880 A
- US 5989835 A
- US 09031271 B
- US 6248516 B
- US 6291158 A
- US 6127197 A
- EP 0368684 A
- US 5270163 A

### Non-patent literature cited in the description

- **IAN FRESHNEY.** Culture of Animal Cells. Wiley-Liss, 1994
- **TAYLOR et al.** *Am. Scientist,* 1992, vol. 80, 322-335
- **A. BARBETTA ; N. R. CAMERON.** *Macromolecules,* 2004, vol. 37, 3188
- **A. S. KABALNOV ; E. G. SHCHUKIN.** *Adv. Colloid Interface. Sci.,* 1992, vol. 38, 69
- Emulsions and Emulsion Technology Part 1. Marcel Dekker Inc, 1974
- **M. P. ARONSON ; M. F. PETKO.** *J. Colloid Interface Sci.,* 1993, vol. 159, 134
- **M. W. HAYMAN ; K. H. SMITH ; N. R. CAMERON ; S. A. PRZYBORSKI.** *Biochem. Biophys. Res. Commun.,* 2004, vol. 314, 483
- **M. W. HAYMAN ; K. H. SMITH ; N. R. CAMERON ; S. A. PRZYBORSKI.** *J. Biochem. Biophys. Methods,* 2005, vol. 62, 231
- **J. G. WEERS.** Modern Aspects of Emulsion Science. RSC, 1998
- **J. C. RAVEY ; M. J. STEBE.** *Prog. Colloid Polym. Sci.,* 1993, vol. 82, 218
- **R. PONS ; P. ERRA ; C. SOLANS ; J. C. RAVEY ; M. J. STEBE.** *J. Phys. Chem.,* vol. 97, 12320
- **R. PONS ; J. C. RAVEY ; S. SAUVAGE ; M. J. STEBE ; P. ERRA ; C. SOLANS.** *Colloids Surf., A: Physicochem. Eng. Aspects,* 1993, vol. 76, 171
- **J. C. RAVEY ; M. J. STEBE ; S. SAUVAGE.** *Colloids Surf., A: Physicochem. Eng. Aspects,* 1994, vol. 91, 237
- **BEIER K ; FAHIMI HD.** Application of automatic image analysis for quantitative morphological studies of peroxisomes in rat liver in conjunction with cytochemical staining with 3-3'-diaminobenzidine and immunocytochemistry. *Microsc Res Tech,* 01 June 1992, vol. 21 (4), 271-282
- **ABU-ABSI SF ; FRIEND JR ; HANSEN LK ; HU WS.** Structural polarity and functional bile canaliculi in rat hepatocyte spheroids. *Exp Cell Res,* 10 March 2002, vol. 274 (1), 56-67
- **BOHNERT A ; HORNUNG J ; MACKENZIE IC ; FUSENIG NE.** Epithelialmesenchymal interactions control basement membrane production and differentiation in cultured and transplanted mouse keratinocytes. *Cell Tissue Res,* 1986, vol. 244, 413-429
- **IKUTA S ; SEKINO N ; HARA T ; SAITO Y ; CHIDA K.** Mouse epidermal keratinocytes in three-dimensional organotypic coculture with termal fibroblasts form a stratified sheet resembling skin. *Biosci Biotechnol Biochem,* 2006, vol. 70, 2669-2675
- **PRUNIERAS M ; REGNIER M ; WOODLEY D.** Methods for cultivation of keratinocytes with an air-liquid interface. *J Invest Dermatol,* 1983, vol. 81, 28s-33s
- **SCHOOP VM ; MIRANCEA N ; FUSENIG NE.** Epidermal organisation and differentiation of HaCaT keratinocytes in organotypic coculture with human dermal fibroblasts. *J Invest Dermatol,* 1999, vol. 112, 343-353